# EUROPEAN PATENT APPLICATION

(11) **EP 4 840 417 A1**
(43) Date of publication of application: **18.11.2026**
(21) Application number: 25305679.0
(22) Date of filing: 13.05.2025
(51) Int. Cl.: A61B 34/10, A61B 34/00, G06N 20/00, G16H 20/40, G16H 30/40, G16H 50/50

(54) **SURGICAL PLANNING TOOL**

(71) Applicant: Sofradim Production, 01600 Trevoux (FR); DIGITAL SURGERY LIMITED, London EC1V 2TT (GB)
(72) Inventor: LE RUYET, Anicet, 01600 Trevoux (FR); JOURDAN, Arthur, 01600 Trevoux (FR); VEGLEUR, Anthony, 01600 Trevoux (FR)
(74) Representative: Germain Maureau

(57) **Abstract**

Examples described herein provide a computer-implemented method that includes using pre-operative scan data of internal anatomy of a patient to generate a simulation. Metrics of a defect of the internal anatomy of the patient based on the simulation can be generated based on the simulation. Repair parameters to simulate a repair of the defect can be determined based on the metrics of the defect. A surgical plan to perform the repair can be displayed on a user interface.

## Description

### BACKGROUND

The present disclosure relates in general to computing technology and relates more particularly to computing technology for surgical procedure planning.

Outcomes from surgical procedures can be impacted by surgical techniques and materials selected for use. Some types of surgical procedures may have limited pre-operative information available depending on the ability to scan and/or predict conditions of anatomy and/or physiology within a patient's body that may not be readily visible through scanning techniques. For example, general region information may be observed through computed tomography (CT) or ultrasound scans, but aspects such as tissue thickness and characteristics of surrounding anatomy may not be readily observable prior to beginning an operation. As a result, a patient may end up with a surgical repair combined with materials which are oversized or undersized for the actual needs of the patient.

### SUMMARY

According to an aspect, a computer-implemented method is provided. The method includes receiving pre-operative scan data of internal anatomy of a patient, generating a simulation of the internal anatomy of the patient based on the pre-operative scan data, and quantifying a plurality of metrics of a hernia and the internal anatomy of the patient based on the simulation. The computer-implemented method also includes determining a plurality of repair parameters to simulate a repair of the hernia using a mesh and a plurality of fixations based on the metrics of the hernia and the internal anatomy. A surgical plan is displayed to perform the repair by positioning the mesh within the internal anatomy and applying the fixations.

According to another aspect, a system includes a memory system and a processing system. The processing system is coupled to the memory system and configured to execute instructions to perform a plurality of operations. The operations include receiving pre-operative scan data of internal anatomy of a patient, the pre-operative scan data including data from one or more scans. The operations also include generating a simulation of the internal anatomy of the patient based on the pre-operative scan data by combining the data from two or more views of the one or more scans and quantifying a plurality of metrics of a defect of the internal anatomy of the patient based on the simulation. The operations further include determining a plurality of repair parameters to simulate a repair of the defect based on the metrics of the defect and displaying a surgical plan to perform the repair.

According to a further aspect, a computer program product is provided. The computer program product includes a memory device having computer executable instructions stored thereon, which when executed by one or more processors cause the one or more processors of a computing system to perform a plurality of operations. The operations include receiving pre-operative scan data of internal anatomy of a patient, generating a simulation of the internal anatomy of the patient based on the pre-operative scan data, and quantifying a plurality of metrics of a defect of the internal anatomy of the patient based on the simulation. The operations additionally include determining a plurality of repair parameters to simulate a repair of the defect based on the metrics of the defect and transmitting or displaying a surgical plan to perform the repair.

The above features and advantages, and other features and advantages, of the disclosure are readily apparent from the following detailed description when taken in connection with the accompanying drawings.

### BRIEF DESCRIPTION OF THE DRAWINGS

The specifics of the exclusive rights described herein are particularly pointed out and distinctly claimed in the claims at the conclusion of the specification. The foregoing and other features and advantages of the aspects of the disclosure are apparent from the following detailed description taken in conjunction with the accompanying drawings in which:
FIG. 1 depicts a computer-assisted surgery (CAS) system according to one or more aspects;
FIG. 2 depicts a surgical procedure system according to one or more aspects;
FIG. 3 depicts a system for analyzing data according to one or more aspects;
FIG. 4 depicts a data loading interface of a surgical planning tool according to one or more aspects;
FIG. 5 depicts an image viewing interface of a surgical planning tool according to one or more aspects;
FIG. 6 depicts a 3D image viewing interface of a surgical planning tool according to one or more aspects;
FIG. 7A depicts a defect viewing interface of a surgical planning tool according to one or more aspects;
FIG. 7B depicts a defect viewing interface of a surgical planning tool with related structure viewing according to one or more aspects;
FIG. 8 depicts a modified defect viewing interface of a surgical planning tool according to one or more aspects;
FIG. 9 depicts a component separation viewing interface of a surgical planning tool according to one or more aspects;
FIG. 10A depicts a repair sizing interface of a surgical planning tool with a first set of parameters according to one or more aspects;
FIG. 10B depicts a repair sizing interface of a surgical planning tool with a second set of parameters according to one or more aspects;
FIG. 11A depicts a simulation interface of a surgical planning tool with a first set of parameters according to one or more aspects;
FIG. 11B depicts a simulation interface of a surgical planning tool with a second set of parameters according to one or more aspects;
FIG. 12 depicts a case selection interface of a surgical planning tool according to one or more aspects;
FIG. 13 depicts a case creation interface of a surgical planning tool according to one or more aspects;
FIG. 14 depicts an image viewing interface of a surgical planning tool with a history viewing interface according to one or more aspects;
FIG. 15 depicts an image viewing interface of a surgical planning tool with a chat interface according to one or more aspects;
FIG. 16 depicts an image viewing interface of a surgical planning tool with a model information interface according to one or more aspects;
FIG. 17 depicts a defect viewing interface of a surgical planning tool according to one or more aspects;
FIG. 18 depicts a case export interface of a surgical planning tool according to one or more aspects;
FIG. 19 depicts a batch creation interface of a surgical planning tool according to one or more aspects;
FIG. 20 depicts a batch results interface of a surgical planning tool according to one or more aspects;
FIG. 21 depicts a flowchart of a surgical planning according to one or more aspects; and
FIG. 22 depicts a block diagram of a computer system according to one or more aspects.

The diagrams depicted herein are illustrative. There can be many variations to the diagrams and/or the operations described herein without departing from the spirit of the described aspects. For instance, the actions can be performed in a differing order, or actions can be added, deleted, or modified. Also, the term "coupled" and variations thereof describe having a communications path between two elements and do not imply a direct connection between the elements with no intervening elements/connections between them. All of these variations are considered a part of the specification.

### DETAILED DESCRIPTION

Exemplary aspects of the technical solutions described herein include systems and methods for a surgical planning tool for surgical procedures. Aspects can generate simulations and surgical planning information for better patient outcomes during a surgical procedure and post-operatively using pre-operative scan data and surgical material data. In the context of a hernia repair, such as a ventral hernia repair, lower quality outcomes may be attributed in part to a lack of consensus on the surgical strategy to adopt for hernia repair. Some surgical strategies may overlook certain patient parameters, such as the biomechanical behavior of the abdominal wall. Pre-operative data, such as CT scan data, may support determining patient information that can be used to derive metrics correlated to reduction of post-operative complications and compare potential surgical outcomes. With respect to a hernia, data from a pre-operative CT scan can be used to determine parameters, such as hernia volume and muscle thickness. According to aspects, a surgical planning tool can support uploading of pre-operative CT scan data and generate a 3D reconstruction of the anatomy of the patient for visualization and further simulations. The 3D reconstruction can be used to derive metrics and compare possible surgical outcomes using repair materials of various sizes and attachments. The surgical planning tool can use the reconstructed geometry to generate mechanical simulations of the potential impact of different repair modalities (e.g., mesh type, size, fixation) so that the surgeon can identify the best surgical procedure for a given patient. Although described herein with reference to CT scan data, the scan data can be any type of pre-operative imaging, such as scan data from CT scanners, ultrasound scanners, X-ray devices, magnetic resonance imaging scanners, and/or other such technologies known in the art.

Aspects of the technical solutions herein are rooted in computing technology, particularly utilizing scan data to generate geometrically accurate simulations of real-world conditions. Aspects can provide a practical application of generating predicted surgical outcome based on simulated characteristics of real-world anatomical structures and surgical repair materials. The insights generated through the surgical planning tool can improve patient outcomes by considering patient specific parameters of defects to be repaired, the condition of surrounding anatomical structures, and characteristics of the repair materials along with methods of attachment. Merging multiple data sources can allow for generation of overlays and simulations that predict patient outcomes before performing a surgical procedure. Further, surgical planning data can be presented during a surgical procedure to provide real-time guidance based on the previously computed simulation data. Such simulations cannot reasonably be performed in the human mind or using pen and paper due to the high volume of data to accurately render simulations as well as the ability to acquire scan data of internal anatomy and interpret the scan data with a high degree of precision in multiple dimensions, orientations, and views. Surgical planning provided through a surgical planning tool can be used, for example, to estimate the impact of some surgical parameters, including the properties of an implant, the potential quality of a repair, and/or other such factors, thereby allowing a surgeon to increase the chance of a successful outcome.

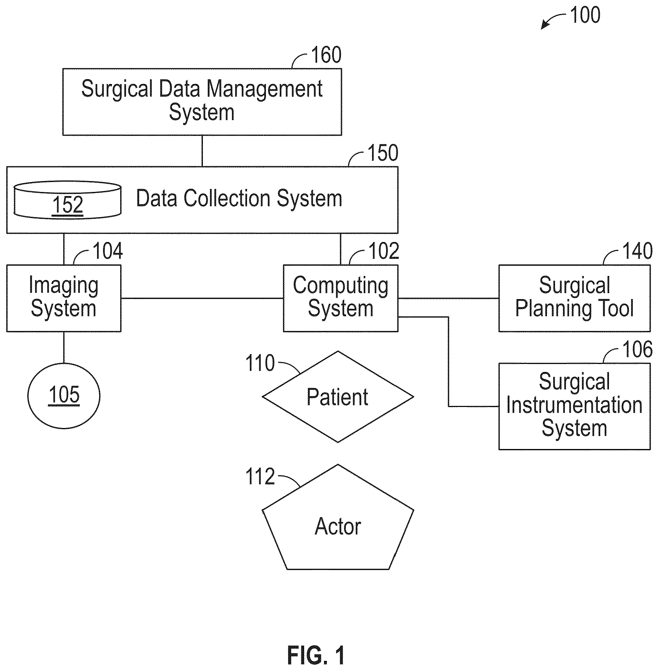

Turning now to FIG. 1, an example computer-assisted surgery (CAS) system 100 is generally shown in accordance with one or more aspects. The CAS system 100 includes at least a computing system 102, an imaging system 104, a surgical instrumentation system 106, and a surgical planning tool 140. As illustrated in FIG. 1, an actor 112 can be medical personnel that uses the CAS system 100 to perform pre-operative planning and/or perform a surgical procedure on a patient 110. Medical personnel can be a surgeon, assistant, nurse, administrator, or any other actor that interacts with the CAS system 100 in a surgical environment. The surgical procedure can be any type of surgery. In other examples, actor 112 can be a technician, an administrator, an engineer, or any other such personnel that interacts with the CAS system 100. For example, actor 112 can record data from the CAS system 100, configure/update one or more attributes of the CAS system 100, review past performance of the CAS system 100, repair the CAS system 100, and/or the like including combinations and/or multiples thereof.

A surgical procedure can include multiple phases, and each phase can include one or more surgical actions. A "surgical action" can include an incision, a compression, a stapling, a clipping, a suturing, a cauterization, a sealing, or any other such actions performed to complete a phase in the surgical procedure. A "phase" represents a surgical event that is composed of a series of steps (e.g., closure). A "step" refers to the completion of a named surgical objective (e.g., hemostasis). During each step, certain surgical instruments (e.g., forceps) are used to achieve a specific objective by performing one or more surgical actions. In addition, a particular anatomical structure of the patient may be the target of the surgical action(s).

The imaging system 104 includes one or more imaging devices 105, such as CT scanners, ultrasound scanners, X-ray devices, magnetic resonance imaging scanners, cameras, and/or the like including combinations and/or multiples thereof. The imaging devices 105 can capture image data and/or video data of pre-operative conditions, post-operative conditions, and/or conditions while the surgical procedure being performed. The imaging system 104 can include imaging devices 105 that are used externally and/or are passed inside of the patient 110.

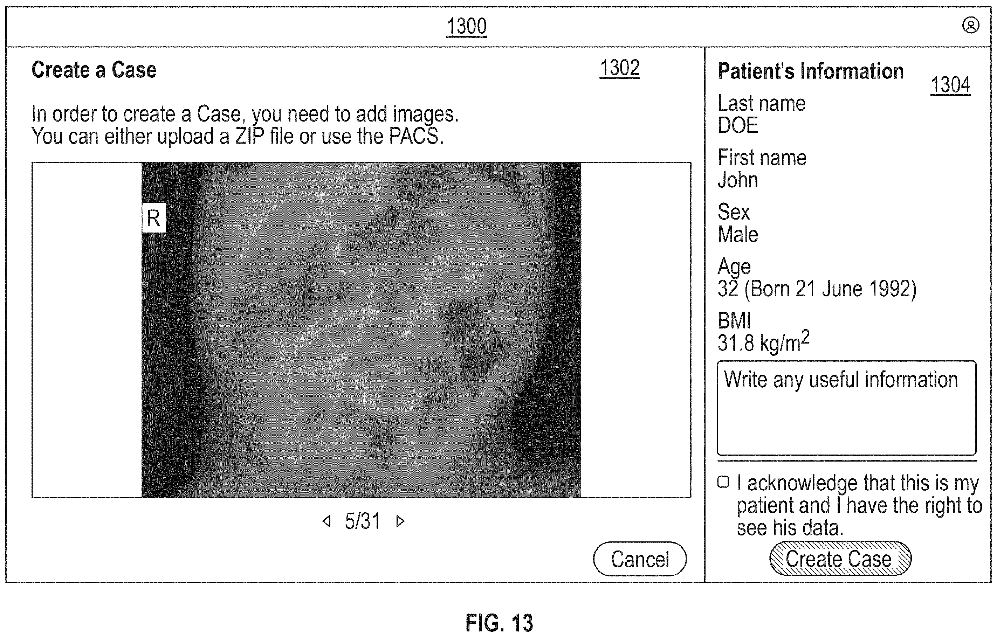

The computing system 102 includes one or more memory devices, one or more processors, a user interface device, among other components. All or a portion of the computing system 102 shown in FIG. 1 can be implemented for example, by all or a portion of computer system 1300 of FIG. 13. Computing system 102 can execute one or more computer-executable instructions. The execution of the instructions facilitates the computing system 102 to perform one or more methods, including those described herein. The computing system 102 can communicate with other computing systems via a wired and/or a wireless network.

The surgical planning tool 140 can include instructions that are executed by the computing system 102. The surgical planning tool 140 can be accessible by the actor 112 while interacting with the patient 110 and/or can be accessed later/remotely while the patient 110 is not physically present.

A data collection system 150 can be employed to store pre-operative, post-operative, and surgical data, including the data captured before, during, and after the surgical procedure. The data collection system 150 includes one or more storage devices 152. The data collection system 150 can be a local storage system, a cloud-based storage system, or a combination thereof. Further, the data collection system 150 can use any type of cloud-based storage architecture, for example, public cloud, private cloud, hybrid cloud, and/or the like including combinations and/or multiples thereof. In some examples, the data collection system can use a distributed storage, i.e., the storage devices 152 are located at different geographic locations. The storage devices 152 can include any type of electronic data storage media used for recording machine-readable data, such as semiconductor-based, magnetic-based, optical-based storage media, and/or the like including combinations and/or multiples thereof. For example, the data storage media can include flash-based solid-state drives (SSDs), magnetic-based hard disk drives, magnetic tape, optical discs, and/or the like including combinations and/or multiples thereof.

In one or more examples, the data collection system 150 can be part of the imaging system 104, or vice-versa. In some examples, the data collection system 150, the imaging system 104, and the computing system 102, can communicate with each other via a communication network, which can be wired, wireless, or a combination thereof. The communication between the systems can include the transfer of data (e.g., video data, instrumentation data, and/or the like including combinations and/or multiples thereof), data manipulation commands (e.g., browse, copy, paste, move, delete, create, compress, and/or the like including combinations and/or multiples thereof), data manipulation results, and/or the like including combinations and/or multiples thereof. In one or more examples, the computing system 102 can manipulate the data already stored/being stored in the data collection system 150. Alternatively, or in addition, the computing system 102 can manipulate the data already stored/being stored in the data collection system 150 based on information from the surgical instrumentation system 106. The surgical instrumentation system 106 can include sensors and equipment used to monitor patient condition and to perform aspects of a surgical procedure. The surgical instrumentation system 106 can be accessed pre-operatively as part of surgical planning as well as during a surgical procedure.

In one or more examples, the imaging data captured by the imaging system 104 is stored on the data collection system 150. In some examples, the computing system 102 curates parts of the data being stored on the data collection system 150. In some examples, the computing system 102 filters the imaging data captured by the imaging system 104 before it is stored on the data collection system 150. Alternatively, or in addition, the computing system 102 imaging data the video captured by the imaging system 104 after it is stored on the data collection system 150. For example, scan data can be organized by position/orientation of the patient to establish multiple points of view of internal anatomy and a target defect or region to perform the surgical procedure.

A surgical data management system 160 can provide access to portions of data captured in the data collection system 150, as well as data and records stored in other systems. The surgical data management system 160 can establish user access permissions to patient and surgical data. The surgical data management system 160 can also control access through an interface based on the user access permissions. Access to the surgical data management system 160 can be provided through one or more applications or secure web pages. The surgical data management system 160 can be a stand-alone application, module, and/or an extension of another system. Additional aspects of the surgical data management system 160 can include accessing artificial intelligence (AI)-powered surgical images, video, insights, and analytics. Further aspects of the surgical data management system 160 can include accessing simulation materials that can assist surgeons to prepare, practice, and teach surgical procedures. Further aspects of the surgical data management system 160 can include integrating aspects of equipment in an operating room, surgery planning, rating surgeon performance, and other such features. The surgical data management system 160 can provide access to or be combined with the surgical planning tool 140.

The surgical planning tool 140 can provide browser-based interfaces or an application based interfaces customized to various types of devices, such as personal computers, tablet computers, mobile devices, and/or other such devices. In some aspects, the surgical planning tool 140 can include an image viewer (e.g., a Digital Imaging and Communications in Medicine (DICOM) viewer), measurement tools, and a messaging interface. Further, the surgical planning tool 140 can include visualization and display interfaces for a three-dimensional reconstruction and display of anatomy, such as an abdominal wall, muscle, hernia sac, intestines, and/or other such structures. Additionally, the surgical planning tool 140 can provide interfaces for extraction of metric for surgery planning (e.g., time, materials to be used, etc.) and extraction of metric to support clinical decisions, such as an emergency or likely need of a particular surgical action.

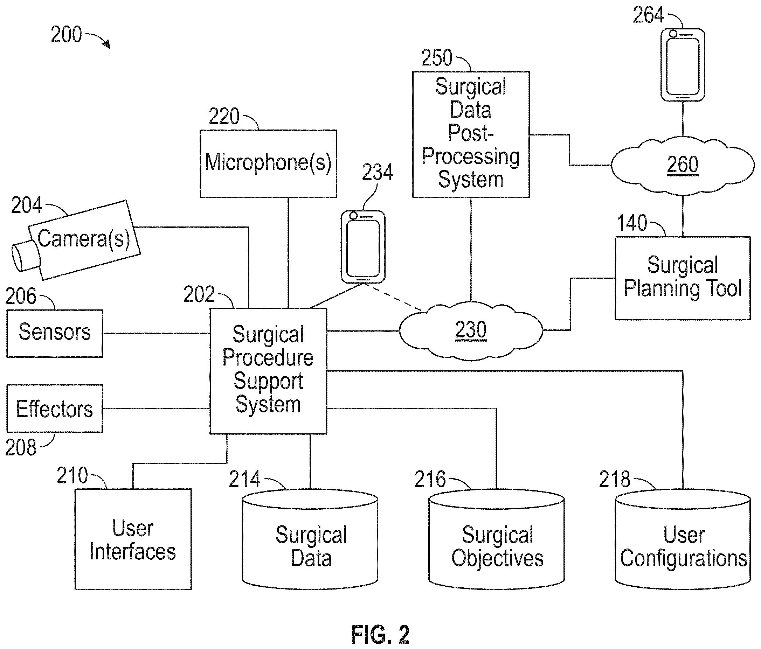

Turning now to FIG. 2, a surgical procedure system 200 is generally shown according to one or more aspects. The example of FIG. 2 depicts a surgical procedure support system 202 that can include or may be coupled to the CAS system 100 of FIG. 1. The surgical procedure support system 202 can acquire image or video data using one or more cameras 204. The surgical procedure support system 202 can also interface with one or more sensors 206 and/or one or more effectors 208. The sensors 206 may be associated with surgical support equipment and/or patient monitoring. The effectors 208 can be robotic components or other equipment controllable through the surgical procedure support system 202. The surgical procedure support system 202 can also interact with one or more user interfaces 210, such as various input and/or output devices. The surgical procedure support system 202 can store, access, and/or update surgical data 214 associated with a training dataset and/or live data as a surgical procedure is being performed on patient 110 of FIG. 1. The surgical procedure support system 202 can store, access, and/or update surgical objectives 216 to assist in training and guidance for one or more surgical procedures. The surgical objectives 216 can include surgical planning information populated through the surgical planning tool 140 for use during a surgical procedure. User configurations 218 can track and store user preferences.

The surgical procedure support system 202 can also communicate with other systems through a network 230. For example, the surgical procedure support system 202 can communicate with the surgical planning tool 140 and a surgical data post-processing system 250 through the network 230. Other types of devices, such as a computing device 234 (e.g., a mobile phone, laptop, personal computer, or tablet computer), can communicate directly with the surgical procedure support system 202 or through the network 230. As one example, user interfaces 210 may be connected to or integrated with the surgical procedure support system 202 by a wired connection while the computing device 234 connects to the surgical procedure support system 202 via a wireless connection. In some aspects, the computing device 234 can execute or link to another computer system that executes the surgical data management system 160 of FIG. 1 to access various data sources through the network 230.

The surgical data post-processing system 250 can receive surgical data and associated data generated by the surgical procedure support system 202 and may be separately stored and secured through other data storage. Access to specific data or portions of data through the surgical data post-processing system 250 may be limited by associated permissions. The surgical data post-processing system 250 may include features such as video viewing, video sharing, data analytics, event data, and selective data extraction.

The surgical planning tool 140 can be used to develop a pre-operative surgical plan and to determine whether changes to the pre-operative surgical plan occurred during a surgical procedure based on various data sources, such as surgical data 214, data collected in the one or more storage devices 152 of FIG. 1, and post-processed data generated by the surgical data post-processing system 250. For example, surgical instrument data, video data, and artificial intelligence generated data can be accessed for viewing and comparison to pre-operative surgical plans through the surgical planning tool 140. The surgical data post-processing system 250 may generate surgical performance metrics and comparison data across data sets collected at multiple locations, making analytics data available to the surgical planning tool 140 to support future planning and post-operative review of a surgical plan. Effects of modifications to a surgical plan developed through the surgical planning tool 140 can be simulated using the surgical planning tool 140. In some aspects, the surgical planning tool 140 and/or surgical data post-processing system 250 can be components of the surgical data management system 160 of FIG. 1.

Surgical planning information generated by the surgical planning tool 140 can be provided to the surgical procedure support system 202, for instance, to store and use as part of the surgical objectives 216. The surgical planning information can be used by an intraoperative assistance interface (e.g., as part of user interfaces 210) showing the surgeon where a mesh should be placed, where an incision should be made, or other such information based on the patient anatomy in real-time. The information can also indicate that some incisions may have to be done in another location because nerves/vessels have been identified. Intraoperative assistance provided through the surgical procedure support system 202 can access additional information sources (e.g., surgical data 214), and use registration processing to combine camera-data from cameras 204 with in-depth (e.g., scan) information.

One or more computing device 264 (e.g., a mobile phone, laptop, personal computer, or tablet computer), can execute the surgical data management system 160 of FIG. 1 to access various data sources through a network 260. The network 230 may be within a facility or multiple facilities maintained within a private network. The network 260 may be a wider area network, such as the internet. Accordingly, the networks 230 and 260 may have access to different files and data sets along with shared access to select files and data sets. In some aspects, networks 230 and 260 can be combined.

According to aspects, the surgical data management system 160 of FIG. 1 can provide multiple user interfaces and functions through the surgical planning tool 140. The user interfaces can include scan data, 3D projections, pre-operative surgical metrics, multiple observation states, component analysis, sizing, simulations, and other such interfaces with supporting analytics.

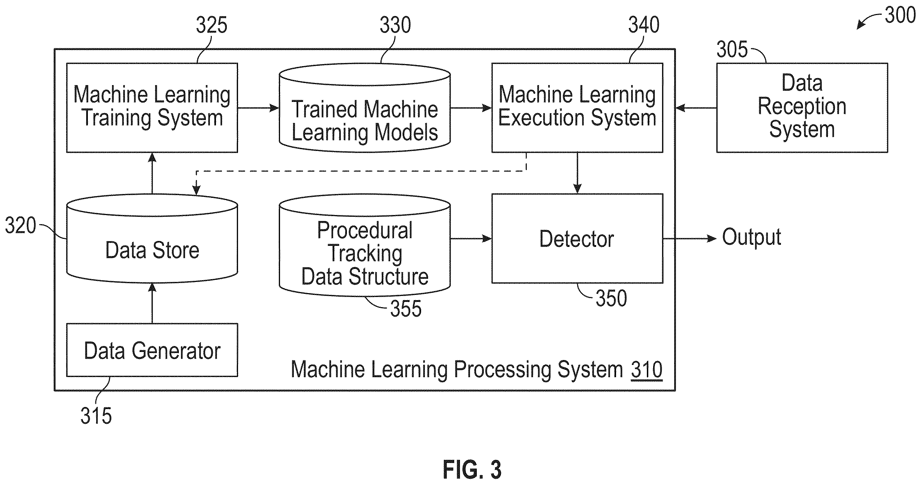

Turning now to FIG. 3, a system 300 for analyzing data is generally shown according to one or more aspects. In accordance with aspects, imaging data can be captured from imaging system 104 of FIG. 1. Data can also be captured by the surgical instrumentation system 106 of FIG. 1. The analysis can result in predicting features that include anatomical structures and surgical materials (e.g., anatomical structure to be repaired, surrounding/proximate anatomical structures, implantable surgical materials, fixations, and/or the like including combinations and/or multiples thereof) associated with the data using machine learning. Other models can be trained to identify conditions associated with surgical devices and/or surgical phases and need not use imaging data. System 300 can be the computing system 102 of FIG. 1, or a part thereof in one or more examples. System 300 uses data streams in the surgical data to identify anatomical features according to some aspects. Further, the system 300 can be implemented as part of the surgical planning tool 140 of FIG. 1 for pre-operative planning. Additionally, some machine learning aspects can be implemented as real-time analysis while other machine learning aspects can be performed as part of post-operative analysis.

System 300 includes a data reception system 305 that collects data, including imaging data and/or surgical instrumentation data. The data reception system 305 can include one or more devices (e.g., one or more user devices and/or servers) located within and/or associated with a medical facility and/or control center. The data reception system 305 can receive surgical data as the data becomes available (e.g., during a scanning/imaging session). Alternatively, or in addition, the data reception system 305 can receive or access surgical data in an offline manner, for example, by accessing data that is stored in the data collection system 150 of FIG. 1.

System 300 further includes a machine learning processing system 310 that processes the data using one or more machine learning models to identify one or more features, such as anatomical structures to be repaired/operated upon, supporting or related structures, and/or the like including combinations and/or multiples thereof, in the imaging data. It will be appreciated that machine learning processing system 310 can include one or more devices (e.g., one or more servers), each of which can be configured to include part or all of one or more of the depicted components of the machine learning processing system 310. In some instances, a part or all of the machine learning processing system 310 is cloud-based and/or remote from an operating room and/or physical location corresponding to a part or all of data reception system 305. It will be appreciated that several components of the machine learning processing system 310 are depicted and described herein. However, the components are just one example structure of the machine learning processing system 310, and that in other examples, the machine learning processing system 310 can be structured using a different combination of the components. Such variations in the combination of the components are encompassed by the technical solutions described herein.

The machine learning processing system 310 can include a machine learning training system 325, which can be a separate device (e.g., server) that stores its output as one or more trained machine learning models 330. The machine learning models 330 are accessible by a machine learning execution system 340. The machine learning execution system 340 can be separate from the machine learning training system 325 in some examples. In other words, in some aspects, devices that "train" the models are separate from devices that "infer," i.e., perform real-time processing of surgical data using the trained machine learning models 330. In some aspects, outputs of the machine learning execution system 340 can be captured in the data store 320 to provide additional data for use by the machine learning training system 325 to revise and update the trained machine learning models 330.

Machine learning processing system 310, in some examples, further includes a data generator 315 to generate simulated surgical data, such as a set of synthetic device data, in combination with real data from the data collection system 150 of FIG. 1, as a set of training data used to generate trained machine learning models 330. Data generator 315 can access (read/write) a data store 320 to record data including multiple data sets. The data sets can be collected before, during, and after one or more procedures (e.g., one or more surgical procedures). For example, the device data may have been collected before, during, or after previous surgeries performed by the actor 112 of FIG. 1 (e.g., surgeon), and the device data can be correlated with recorded imaging data, for instance, from pre-operative and post-operative CT scans of a patient 110 of FIG. 1. The data store 320 is separate from the data collection system 150 of FIG. 1 in some examples. In other examples, the data store 320 is part of the data collection system 150.

Each data set in the data store 320 used as a set of training data for performing training (e.g., generating the machine learning models 330) can be defined as a set of conditions and can be associated with other data that characterizes anatomical structures. Other data can also identify a type of procedure, devices used in the procedure, surgical objectives, and/or an outcome of the procedure. Alternatively, or in addition, the other data can indicate a stage of the procedure with which device data corresponds and device configuration to assist in simulating likely outcomes of surgical procedures. Further, the other data can include data that identifies and/or characterizes one or more objects (e.g., tools, anatomical objects, and/or the like including combinations and/or multiples thereof) with which a device interacts. The characterization can indicate position, orientation, or usage parameters. For example, the characterization can indicate a set of data that corresponds to a usage of a surgical tool and/or a state of the surgical tool resulting from a past or current user handling. Localization can be performed using a variety of techniques for identifying features in one or more coordinate systems.

The machine learning training system 325 can use the recorded data in the data store 320, which can include the simulated surgical data (e.g., set of synthetic device performance parameters) and/or actual surgical data to generate the trained machine learning models 330. The trained machine learning models 330 can be defined based on a type of model and a set of hyperparameters (e.g., defined based on input from a client device). The trained machine learning models 330 can be configured based on a set of parameters that can be dynamically defined based on (e.g., continuous or repeated) training (i.e., learning, parameter tuning). Machine learning training system 325 can use one or more optimization algorithms to define the set of parameters to minimize or maximize one or more loss functions. The set of (learned) parameters can be stored as part of the trained machine learning models 330 using a specific data structure for a particular trained machine learning model of the trained machine learning models 330. The data structure can also include one or more non-learnable variables (e.g., hyperparameters and/or model definitions).

Machine learning execution system 340 can access the data structure(s) of the trained machine learning models 330 and accordingly configure the trained machine learning models 330 for inference (e.g., prediction, classification, and/or the like including combinations and/or multiples thereof). The trained machine learning models 330 can include, for example, a fully convolutional network adaptation, an adversarial network model, an encoder, a decoder, or other types of machine learning models. Further examples of the trained machine learning models 330 can include regression-based models, decision trees, and/or other such machine-learning approaches. The type of the trained machine learning models 330 can be indicated in the corresponding data structures. The trained machine learning models 330 can be configured in accordance with one or more hyperparameters and the set of learned parameters.

While some techniques for predicting a conditions of anatomical structures and surgical modifications to anatomical structures during a surgical phase ("phase") in the surgical procedure are described herein, it should be understood that any other technique for predictions and simulations can be used without affecting the aspects of the technical solutions described herein. In some examples, the machine learning processing system 310 includes a detector 350 that uses the trained machine learning models 330 to identify various items or states within the surgical procedure ("procedure"). The detector 350 can use a particular procedural tracking data structure 355 from a list of procedural tracking data structures. The detector 350 can select the procedural tracking data structure 355 based on the type of surgical procedure that is being performed. In one or more examples, the type of surgical procedure can be predetermined or input by actor 112. For instance, the procedural tracking data structure 355 can identify a set of potential phases that can correspond to a part of the specific type of procedure as "phase predictions", where the detector 350 may perform phase detection. Similarly, anatomical predictions can include predicting pre-operative conditions of anatomical structures and post-operative results that repair or modify anatomical structures after completing the surgical phases.

In some examples, the procedural tracking data structure 355 can be a graph that includes a set of nodes and a set of edges, with each node corresponding to a potential phase. The edges can provide directional connections between nodes that indicate (via the direction) an expected order during which the phases will be encountered throughout an iteration of the procedure. The procedural tracking data structure 355 may include one or more branching nodes that feed to multiple next nodes and/or can include one or more points of divergence and/or convergence between the nodes. In some instances, a phase indicates a procedural action (e.g., surgical action) that is being performed or has been performed and/or indicates a combination of actions that have been performed. In some instances, a phase relates to a biological state of a patient undergoing a surgical procedure. For example, the biological state can indicate a complication (e.g., blood clots, clogged arteries/veins, and/or the like including combinations and/or multiples thereof), pre-condition (e.g., lesions, polyps, and/or the like including combinations and/or multiples thereof).

Each node within the procedural tracking data structure 355 can identify one or more characteristics of the phase corresponding to that node. The characteristics can include device characteristics. In some instances, the node identifies one or more techniques that are typically performed during the phase. The node can also identify a typical type of movement/usage of a surgical tool. Thus, detector 350 can use the segmented data generated by machine learning execution system 340 that indicates the presence and/or characteristics of particular objects in relation to a surgical tool to identify an estimated node to which the real data relates. Identification of the node (i.e., phase) can further be based upon previously detected phases for a given procedural iteration and/or other detected input.

The detector 350 can output predictions, such as insights associated with a portion of the data that is analyzed by the machine learning processing system 310. The insights are associated with the portion of the data that is analyzed by the machine learning execution system 340. The insights associated with surgical devices can be output as alerts to overlay on video or otherwise notify the actor 112. Further, the insights, in one or more examples, can include additional data dimensions, such as, but not limited to, identities of the structures (e.g., instrument, anatomy, and/or the like including combinations and/or multiples thereof) that are identified by the machine learning execution system 340 in the portion of the data that is analyzed. Further, other types of outputs of the detector 350 can include state information or other information used to generate audio output, visual output, and/or commands. For instance, the output can trigger an alert, an augmented visualization, identify a predicted current condition, identify a predicted future condition, command control of equipment, and/or result in other such data/commands being transmitted to a support system component, e.g., through surgical procedure support system 202 of FIG. 2.

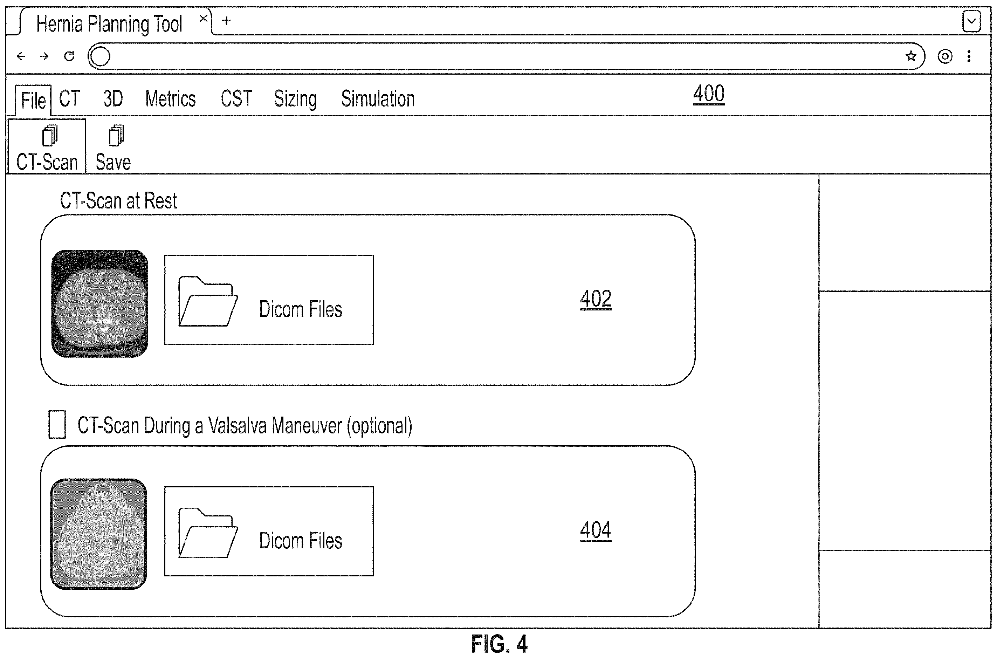

Turning now to FIG. 4, a data loading interface 400 of the surgical planning tool 140 is depicted according to one or more aspects. The data loading interface 400 can include multiple options for loading scan data, such as CT scan data. For example, a first loading interface 402 can support loading of scan data associated with a first state of the patient 110 of FIG. 1, such as an at-rest state. A second loading interface 404 can support loading of scan data associated with a second state of the patient 110, such as an expanded state of the patient 110. For instance, in the context of a hernia surgery, a Valsalva maneuver can be used enhance viewing of the hernia or other such defect. In some aspects, the scan data can be stored and imported as medical images in DICOM file format.

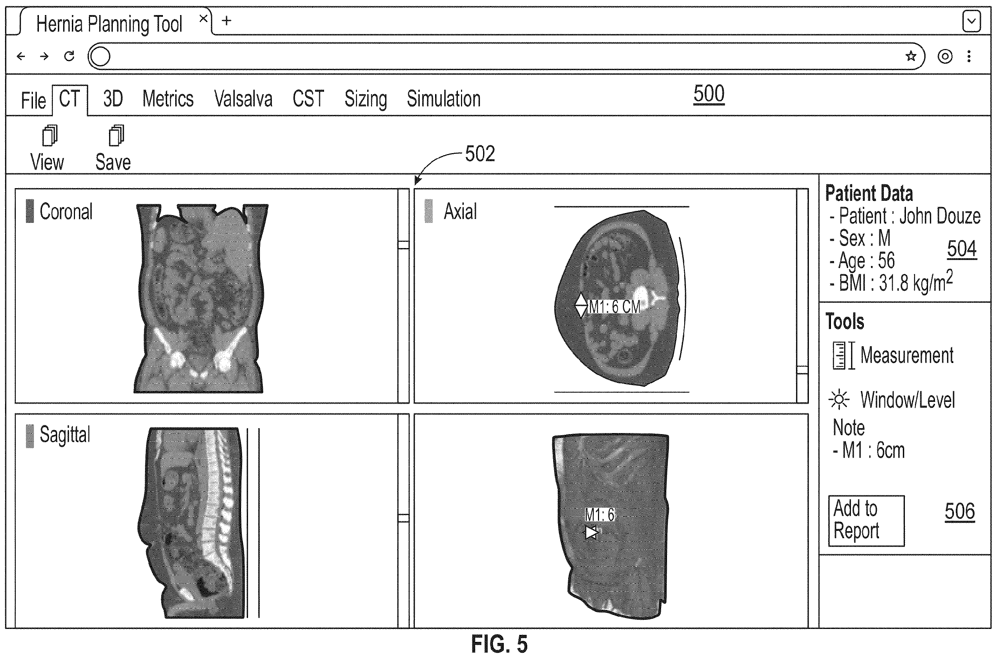

FIG. 5 depicts an image viewing interface 500 of the surgical planning tool 140 according to one or more aspects. The image viewing interface 500 can display scan data loaded through the data loading interface 400 of FIG. 4. The scan data can be depicted in multiple views 502. For example, the views 502 can include one or more of a front view, a rear view, a side view, and an axial view. Further, a projected view can be developed by combining data from the various views 502. The machine learning processing system 310 of FIG. 3 can be trained to process scan data from multiple views and project an anatomical image which may use portions of simulated / approximated anatomical structures to fill in gaps from the scan data acquired through the imaging system 104 of FIG. 1. The image viewing interface 500 can also display patient data 504 and/or a tools interface 506. The patient data 504 can identify the patient 110 and display clinically relevant data. For instance, body mass index data may be used to scale portions of the projected view of anatomy of the patient 110. The patient data 504 may be acquired through accessing electronic medical record data. The tools interface 506 can include user interactive tools, such as a digital measurement tool that can allow a user to measure the size of various anatomical features appearing in the views 502. As one example, upon using the digital measurement tool to measure a feature in one of the views 502, the measurement can be displayed in one or more of the other views where the same feature is visible, e.g., in the projected view. This cross-view measurement display can assist in understanding the orientation and relative size of features of interest from multiple points of view. Other tools can include adjusting the size, brightness, color, contrast, and other such aspects of the views 502, for example.

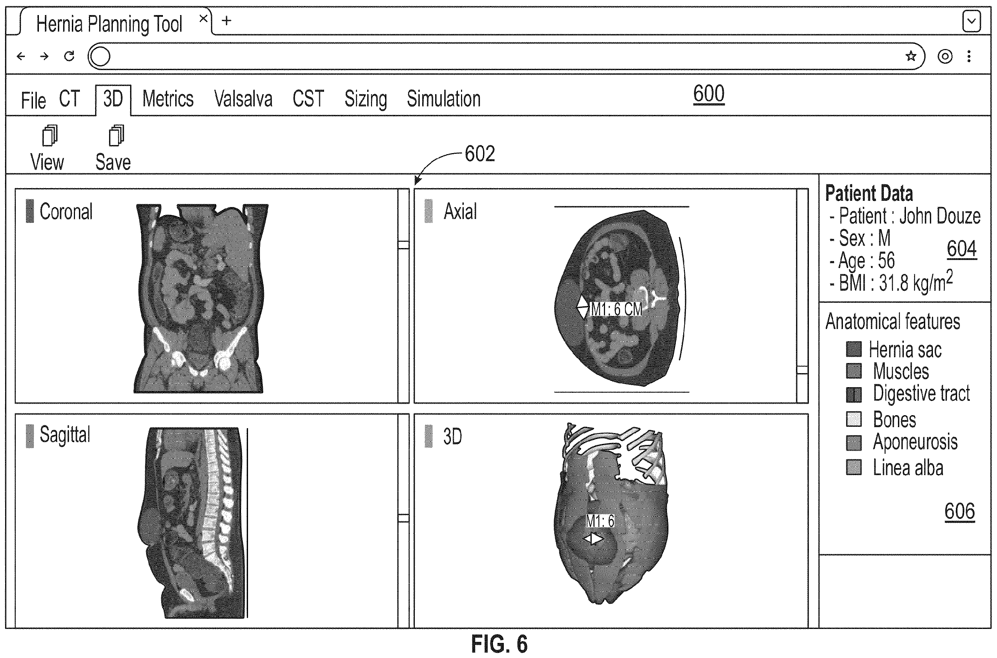

FIG. 6 depicts a 3D image viewing interface 600 of the surgical planning tool 140 according to one or more aspects. The 3D image viewing interface 600 can include a multiple views 602, such as a 3D projected image of internal anatomy of the patient 110 based on other scan-based views of the internal anatomy. The 3D projected image can be generated, for example, by the machine learning processing system 310 of FIG. 3, which may be trained to process scan data from multiple views and project an anatomical image in three dimensions and may use portions of simulated / approximated anatomical structures to fill in gaps from the scan data acquired through the imaging system 104 of FIG. 1. The 3D image viewing interface 600 can also display patient data 604 similar to the patient data 504 of FIG. 5. Various anatomical features 606 can be identified and color coded to support visual identification through the views 602. In some aspects, various layers of anatomical structures can be made visible or not visible to allow the user to study sizing and orientation of various anatomical structures. Further, similar to the example of FIG. 5, digital measurements made through a digital measurement tool can be visible across two or more of the views 602.

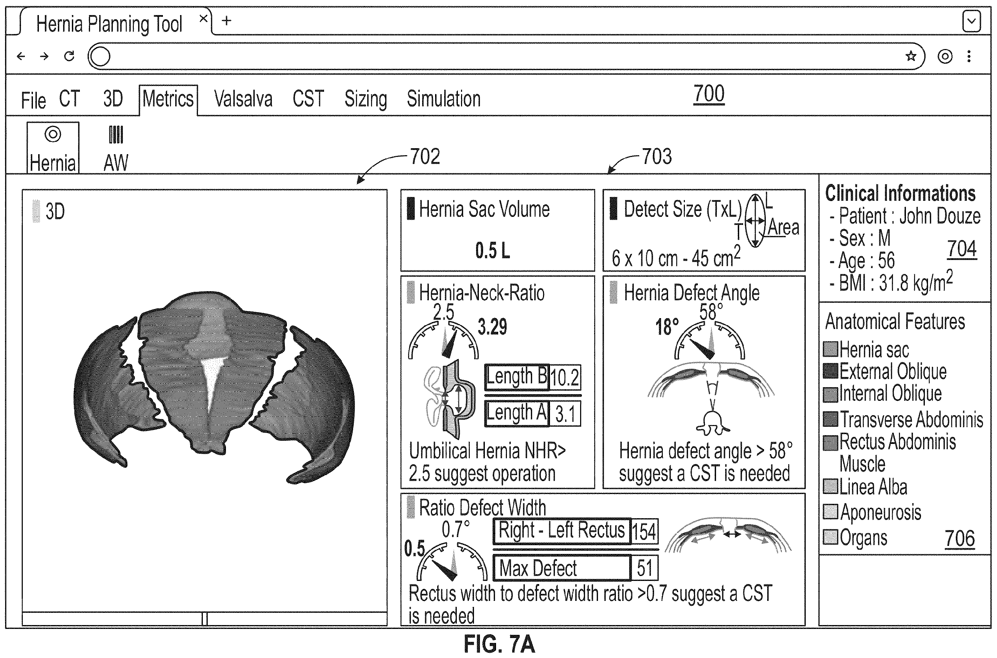

FIG. 7A depicts a defect viewing interface 700 of the surgical planning tool 140 according to one or more aspects. The defect viewing interface 700 can include a view 702 of a defect and associated anatomical structures that may be interactive to rotate, zoom in, zoom out, and the like. The defect viewing interface 700 can also include a metrics display 703 that displays metrics proximate to the view 702 of the internal anatomy. In the context of a hernia, the metrics can include one or more of: a hernia sac volume, a defect size, a hernia-neck ratio, a hernia defect angle, and a ratio defect width. An interactive digital measurement interface of the surgical planning tool 140 can quantify a plurality of metrics of a hernia and the internal anatomy of the patient 110 based on a simulation of the internal anatomy. The view 702 can depict a portion of the simulation of the internal anatomy; however, some underlying parameters of the simulation may not be directly visible. The interactive digital measurement interface can include aspects depicted across multiple user interfaces that allow manual entry of various measurements made through real-world measurements or digital measurements made, for instance, using a measurement tool (e.g., see FIG. 5). Measurements can be in the form of length, width, height, radius, angles, and ratios that compare features such as length ratios (e.g., hernia-neck ratio), width ratios (e.g., ratio defect width), and area/volume computations that can be associated with specific shapes (e.g., elliptical area). Further, defect viewing interface 700 can also display patient data 704 similar to the patient data 504 of FIG. 5 and various anatomical features 706 similar to the anatomical features 606 of FIG. 6.

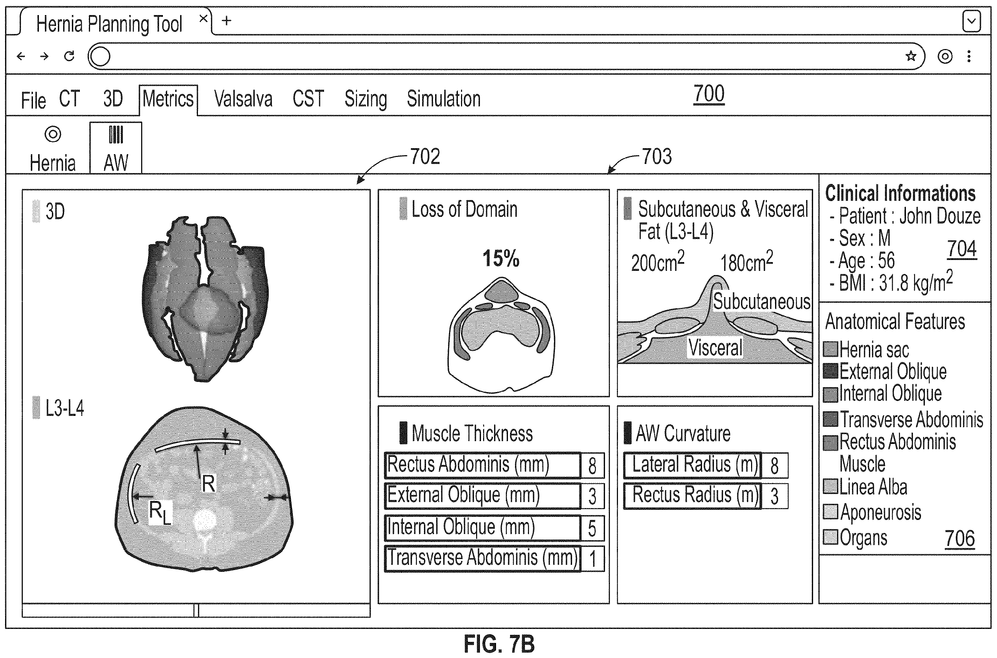

Some metrics may be associated with the defect, such as a hernia, and other metrics may relate to surrounding structures, such as an abdominal wall, as further illustrated in the example of FIG. 7B. For example, the metrics display 703 of FIG. 7B can include one or more of: a volume ratio of fat to abdominal cavity, a fat measurement, a muscle thickness, and an abdominal wall curvature. A volume ratio of fat to abdominal cavity can compare a volume of visceral fat to an abdominal cavity volume. A fat measurement can compute an area or volume of subcutaneous fat and visceral fat proximate to a defect. A muscle thickness can measure muscle thickness at various locations proximate to the defect. An abdominal wall curvature can measure the radius of various portions of an abdominal wall, such as a lateral radius and a rectus radius. While the example of FIGS. 7A and 7B illustrate several possible metrics that can be determined, it will be understood that many variations are possible and specific metrics may depend upon the type of surgical procedure being performed.

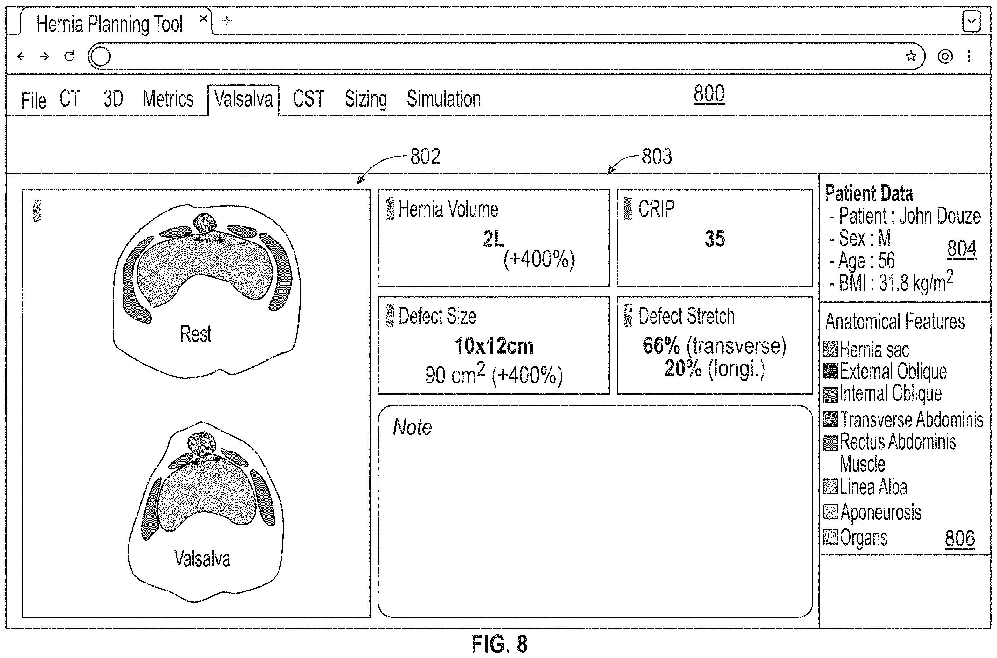

FIG. 8 depicts a modified defect viewing interface 800 of the surgical planning tool 140 according to one or more aspects. The modified defect viewing interface 800 can include a view 802 that displays internal imaging in the at-rest state proximate to the expanded state. For example, pre-operative scan data of internal anatomy of the patient can be received that includes internal imaging in an at-rest state and an expanded state. The expanded state can be an adjustment to the patient 110 that increases visibility of the defect, such as a Valsalva maneuver in the context of a hernia. The modified defect viewing interface 800 can also display metrics 803 including one or more of: a hernia volume change, a critical resistance to impacts related to pressure (CRIP) value, a defect size change, and a defect stretch measurement based on at least one difference between the at-rest state and the expanded state. Change information can be presented in terms of area, volume, and/or percentage change. Further, the modified defect viewing interface 800 can also display patient data 804 similar to the patient data 504 of FIG. 5 and various anatomical features 806 similar to the anatomical features 606 of FIG. 6.

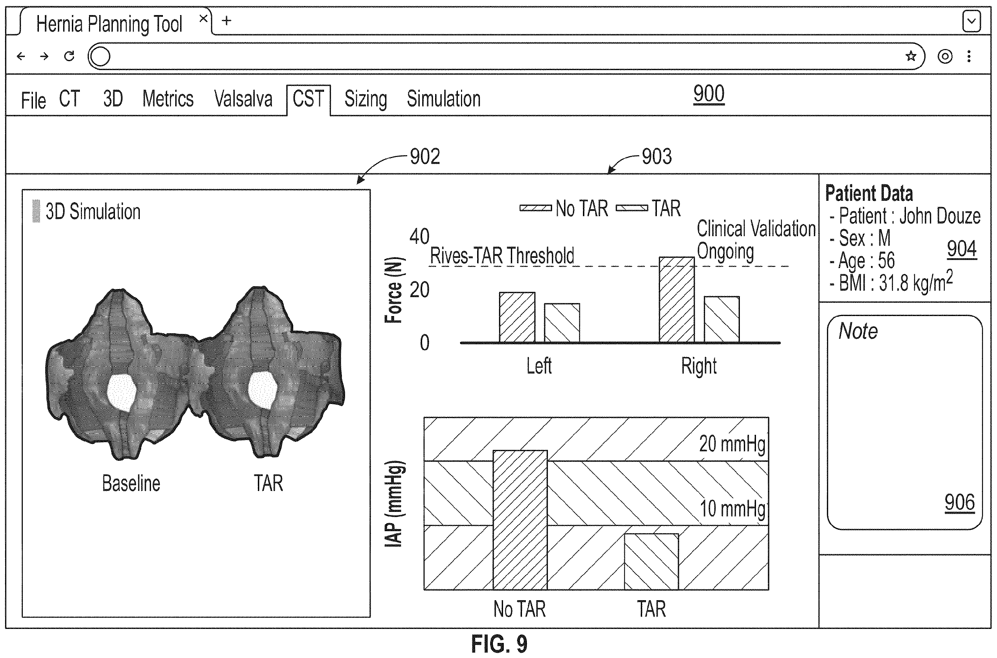

FIG. 9 depicts a component separation viewing interface 900 of the surgical planning tool 140 according to one or more aspects. The component separation viewing interface 900 can include a simulation 902 of a transversus abdominis muscle release (TAR) relative to a baseline in combination with one or more charts 903 illustrating a simulated effect of performing the TAR and not performing the TAR. One or more thresholds can be displayed relative to the charts 903 to visually illustrate whether a threshold condition is exceeded for left or right anatomical portions. The simulation 902 can be illustrated in a 3D view with animation to simulate results of performing the TAR and not performing the TAR. Further, the component separation viewing interface 900 can also display patient data 904 similar to the patient data 504 of FIG. 5 and a notes interface 906 to document observations.

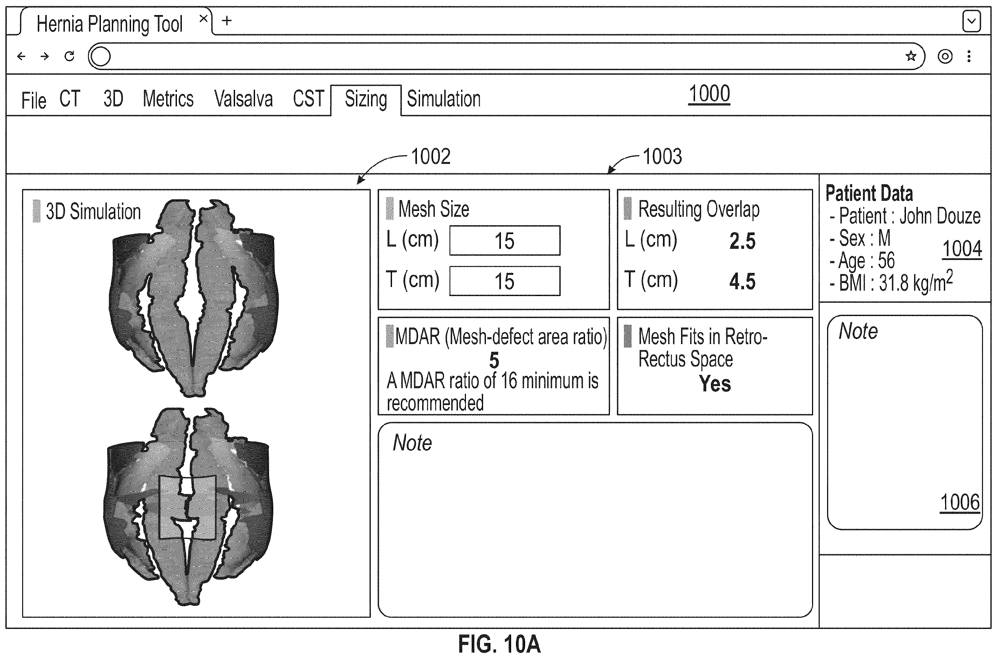

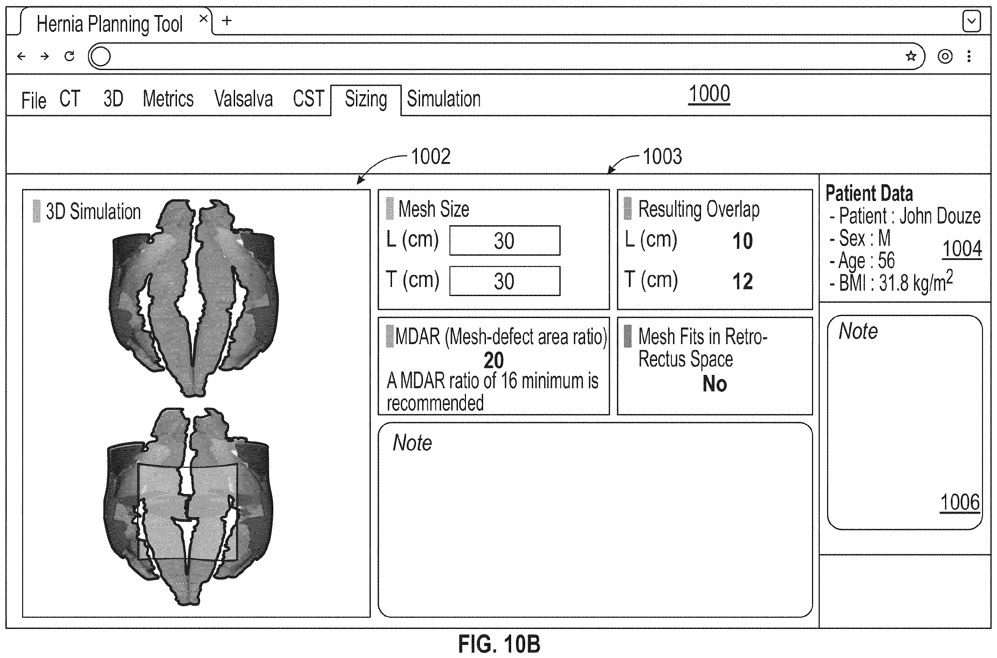

FIG. 10A depicts a repair sizing interface 1000 of the surgical planning tool 140 with a first set of parameters according to one or more aspects. The repair sizing interface 1000 can display a simulation 1002 with a graphical overlay illustrating sizing of the mesh relative to the internal anatomy. The repair sizing interface 1000 can also include a mesh sizing interface 1003 including one or more of: a mesh size, a resulting overlap, a mesh-defect area ratio (MDAR), and an indication of whether the mesh fits in a retro-rectus space of the internal anatomy. As different mesh sizes are selected with sizing information displayed in the mesh sizing interface 1003, the simulation 1002 can be updated visually to illustrate sizing and placement impact as depicted in the example of FIG. 10B. Changes to mesh sizing can also impact values of the resulting overlap, MDAR, and the indication of whether the mesh fits in a retro-rectus space of the internal anatomy. Guidance information can be included to establish suggested limits, such as a minimum recommended MDAR value. Further, the repair sizing interface 1000 can also display patient data 1004 similar to the patient data 504 of FIG. 5 and a notes interface 1006 to document observations.

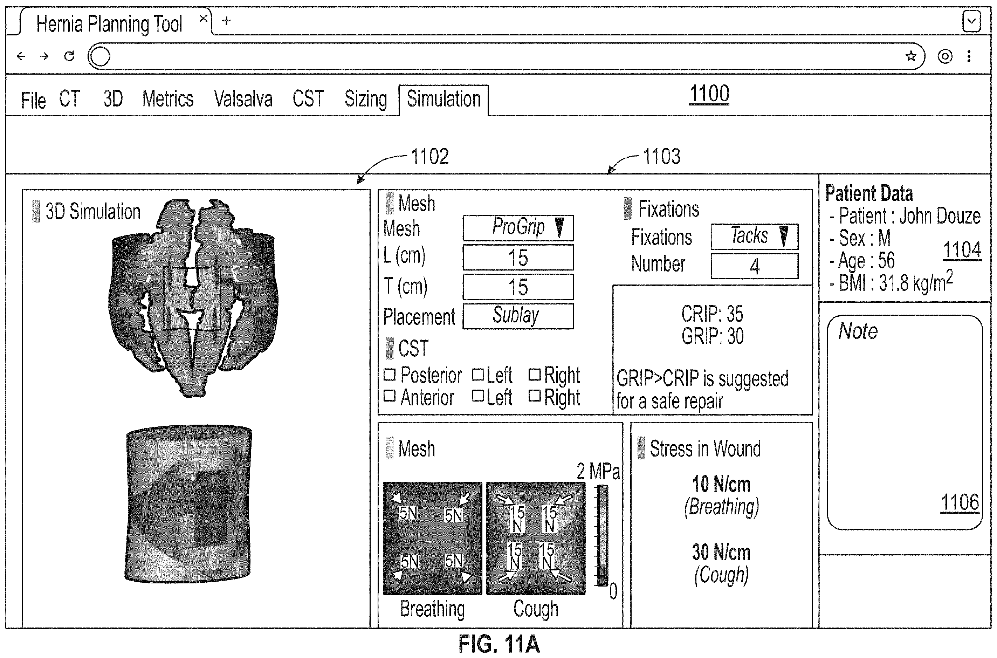

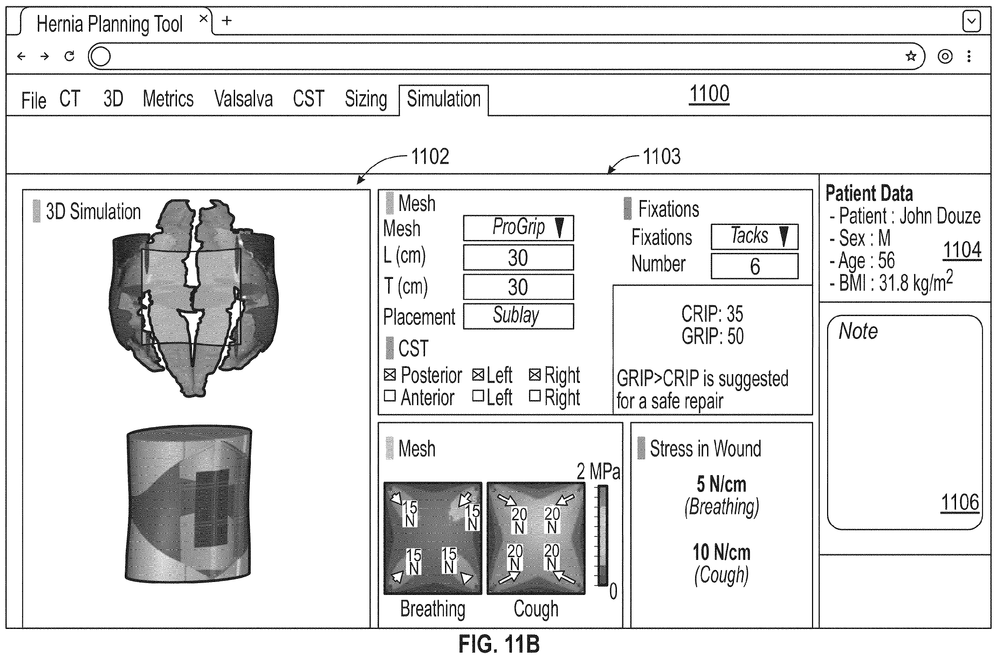

FIG. 11A depicts a simulation interface 1100 of the surgical planning tool 140 with a first set of parameters according to one or more aspects. The simulation interface 1100 can display a surgical plan as a simulation 1102 illustrating mesh and fixation placements. The simulation interface 1100 can also display plurality of repair parameters 1103, such as a mesh type, a mesh size, a mesh placement, a fixation type, and a number of fixations. Modifications can include adjusting between a posterior and an anterior placement with left / right attachments, as depicted, for example, in FIG. 11B. Further, the simulation interface 1100 can also display patient data 1104 similar to the patient data 504 of FIG. 5 and a notes interface 1106 to document observations. The simulation interface 1100 can also display a simulation of stresses placed on the mesh for breathing and coughing conditions in combination with a comparison of a critical resistance to impacts related to pressure (CRIP) value to a gained resistance to impacts related to pressure (GRIP) value for the mesh with the fixations applied. Guidance information can also be displayed, such as verifying that the GRIP value is greater than the CRIP value for a safer repair. The CRIP value can be determined using known techniques, such as multiplying (0.5 * hernia size + 15) * tissue distension. The GRIP value can be computed using known techniques that incorporate an MDAR, mesh, suture and other factors. As one example, the GRIP can be computed as MDAR * coefficients for mesh adhesiveness, mesh position in the abdominal wall, number and type of fixation plus factors for peritoneal and fascial closure. Mesh and wound stresses can be displayed as graphical overlays with heat map depictions and/or as numerical values. The simulation 1102 can be presented in 3D with color coding for anatomical structures and/or stress values. The stress values can change with animation, for instance, to illustrate responses to various events, such as coughing vs. normal breathing. When acceptable size, mesh type, placement, and fixation are determined, the resulting surgical plan can be saved and may be transferred to one or more other systems for use during a surgical procedure. For instance, surgical plan data with visual information can be transferred to the surgical procedure support system 202 of FIG. 2 and saved as part of the surgical objectives 216 for reference during performance of the surgical procedure.

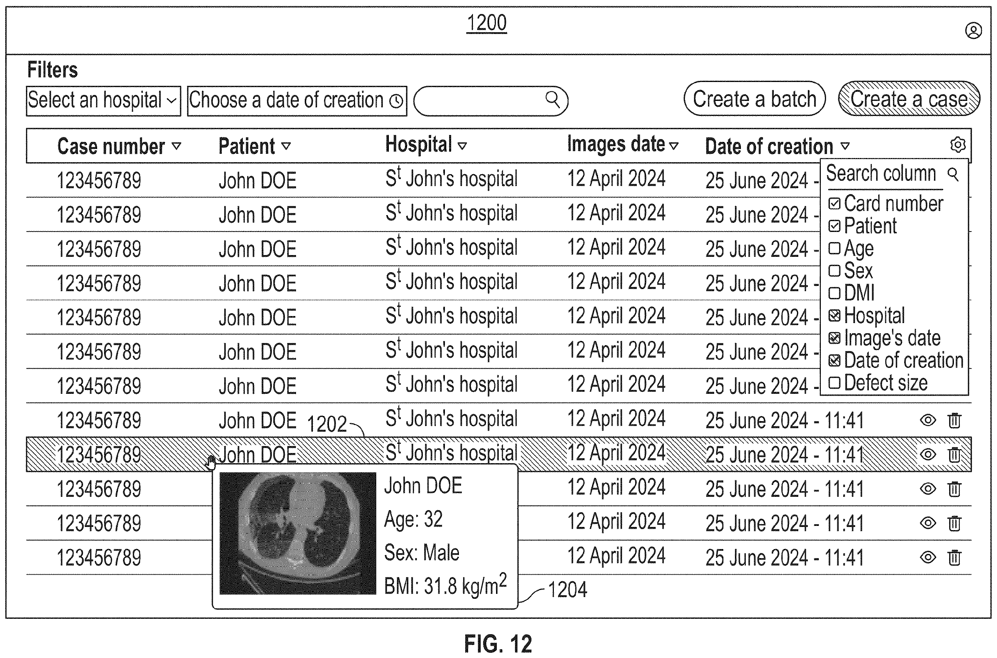

FIG. 12 depicts a case selection interface 1200 of the surgical planning tool 140 according to one or more aspects. The case selection interface 1200 can allow a user to create a single case or a batch. Batch processing can allow data from multiple cases to be analyzed at one time. For example, batch processing can analyze case data from multiple cases to generate metrics and export results. Metrics may have associated ranges or thresholds to distinguish in-range versus potentially out-of-range conditions. When the surgical planning tool 140 is configured with clinically established limits/ranges or customized limits/ranges, batch processing can allow a user to process multiple cases at once and flag cases by the surgical planning tool 140 that may need closer analysis. Batch processing can be performed as a background task to allow a user to continue interacting with the surgical planning tool 140 while metrics processing and data transfer are performed. In some aspects, the surgical planning tool 140 can allow the user to set custom processing constraints for batched data, such as thresholds or limits to flag cases of interest from the batch. The case selection interface 1200 can display information such as case number, patient, age, body mass index, sex, hospital, image date, date of case creation, defect information, and other such information. The case selection interface 1200 can allow a user to view case details, delete a case, or select 1202 a case and view a preview image 1204 with summary information. The case selection interface 1200 can also include a search interface and filtering options, as well as other features.

FIG. 13 depicts a case creation interface 1300 of the surgical planning tool 140 according to one or more aspects. A creation panel 1302 can allow viewing of image data from various file types and sources to associate with a case. A patient information panel 1304 can allow editing of associated patient information.

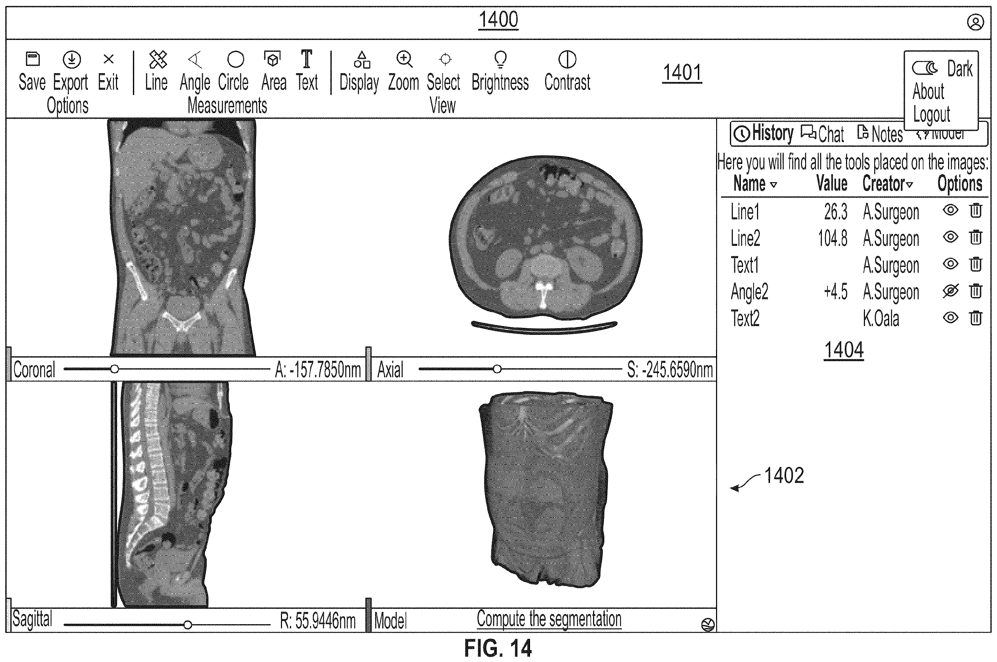

FIG. 14 depicts an image viewing interface 1400 of the surgical planning tool 140 with a history viewing interface 1404 according to one or more aspects. The image viewing interface 1400 can include a menu 1401 to support viewing, measurement, annotation, saving, and exporting of image data in a viewing region 1402. The history viewing interface 1404 can track a history of edits made to annotated images displayed through the image viewing interface 1400. The history viewing interface 1404 can allow a user to see previous versions of annotated images and may allow authorized users to delete/undo annotations, for instance, to revert to a previous version of annotation.

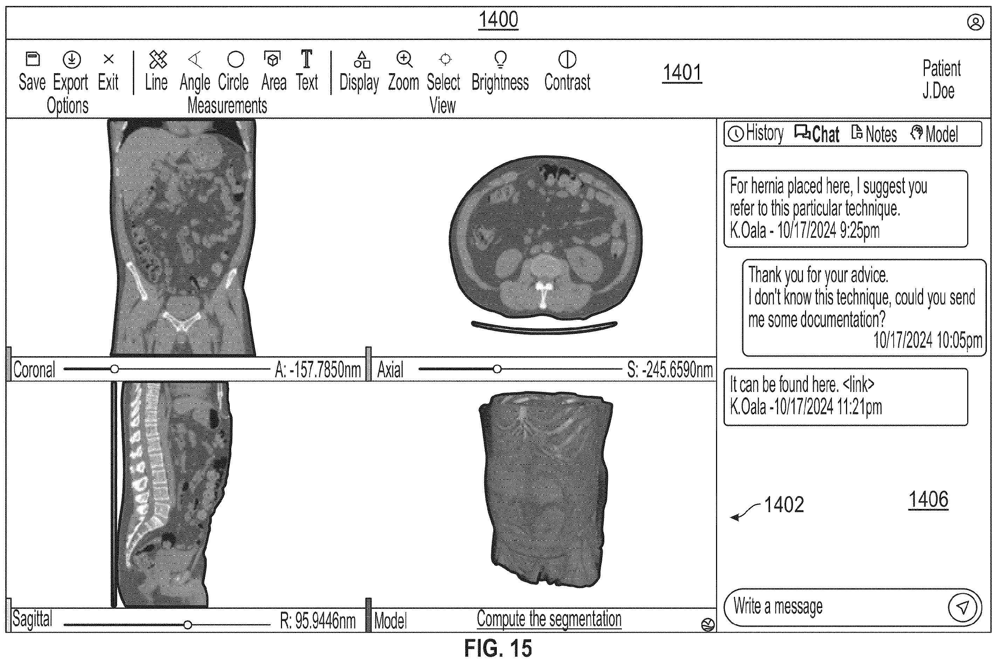

FIG. 15 depicts the image viewing interface 1400 of the surgical planning tool 140 with a chat interface 1406 according to one or more aspects. The chat interface 1406 can allow multiple users the same case to exchange comments and information with each other through a messaging system. In some aspects, the chat interface 1406 can be linked to an external device, such that posting of comments triggers a notification to an associated user. Similarly, the image viewing interface 1400 can support recording of notes related to the case. Data entry can include typed text, images, video clips, audio clips, and/or other such information as content within a chat session or note.

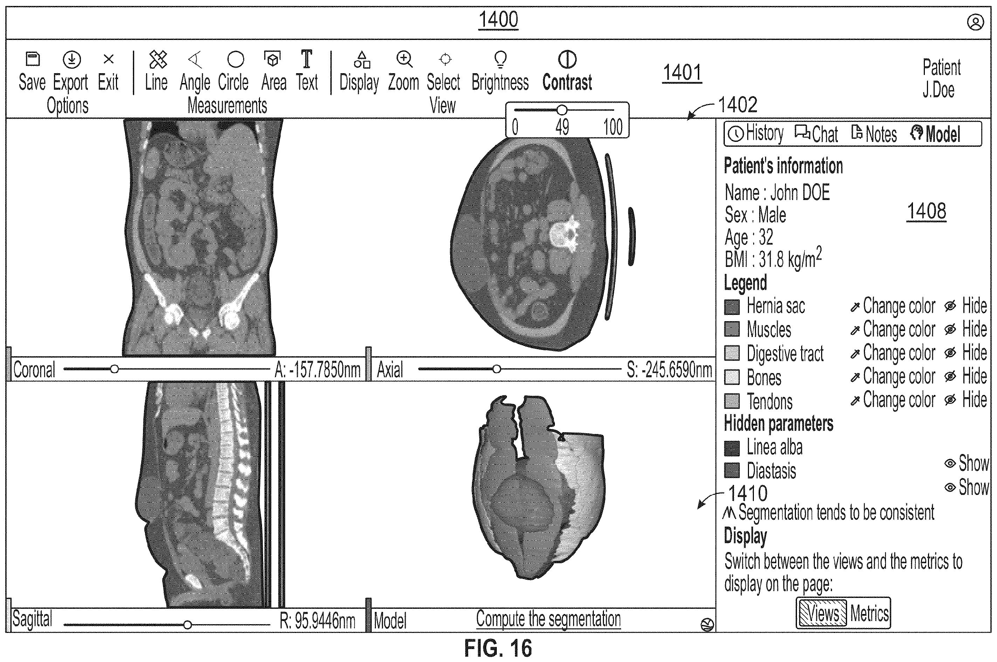

FIG. 16 depicts the image viewing interface 1400 of the surgical planning tool 140 with a model information interface 1408 according to one or more aspects. The model information interface 1408 can document patient information and allow the changing of colors and/or other aspects of a visual model 1410 that can be displayed in one or more views of the viewing region 1402. The images in the visual display can be enhanced through individual zooming in/out, contrast adjustments, sharp/fine visual filtering, annotations, and other such adjustments. Some visual content, such as the visual model 1410, may be rendered in three dimensions and support rotation and orientation adjustments to support multiple points of view.

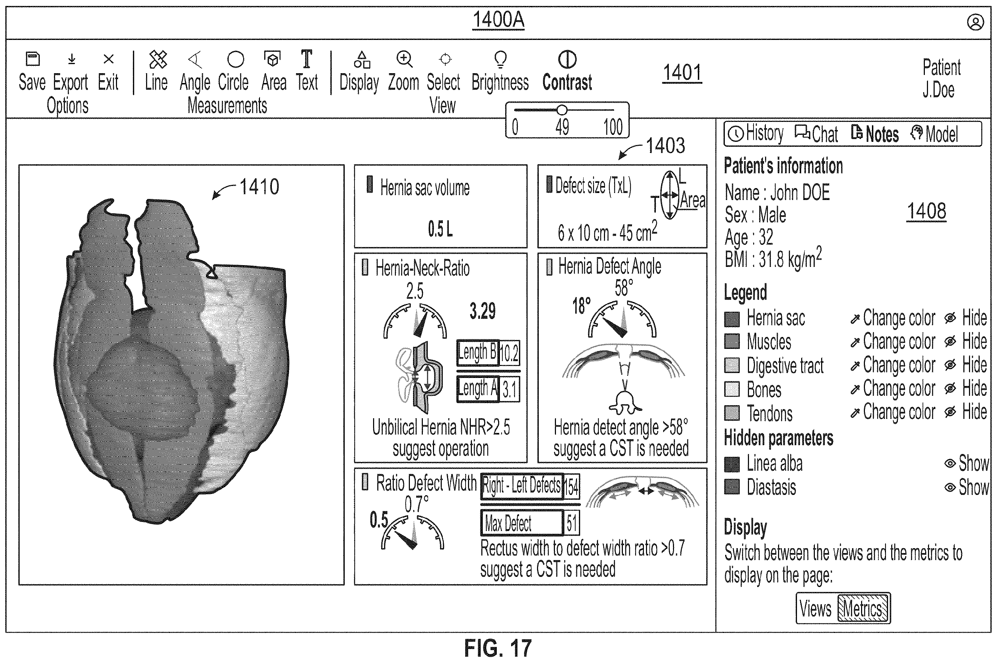

FIG. 17 depicts a defect viewing interface 1400A of the surgical planning tool 140 according to one or more aspects. The defect viewing interface 1400A can be an alternate viewing mode of the image viewing interface 1400 of FIG. 14-16. The defect viewing interface 1400A can display the visual model 1410 in greater detail, illustrating, for example, a defect and associated anatomical structures that may be interactive to rotate, zoom in, zoom out, and the like. The defect viewing interface 1400A can also include a metrics display 1403 that displays metrics proximate to the visual model 1410 of internal anatomy. In the context of a hernia, the metrics can include one or more of: a hernia sac volume, a defect size, a hernia-neck ratio, a hernia defect angle, and a ratio defect width. An interactive digital measurement interface of the surgical planning tool 140 can quantify a plurality of metrics of a hernia and the internal anatomy of the patient 110 based on a simulation of the internal anatomy. The visual model 1410 can depict a portion of the simulation of the internal anatomy; however, some underlying parameters of the simulation may not be directly visible. The interactive digital measurement interface can include aspects depicted across multiple user interfaces that allow manual entry of various measurements made through real-world measurements or digital measurements made, for instance, using a measurement tool (e.g., see FIG. 5). Measurements can be in the form of length, width, height, radius, angles, and ratios that compare features such as length ratios (e.g., hernia-neck ratio), width ratios (e.g., ratio defect width), and area/volume computations that can be associated with specific shapes (e.g., elliptical area). Further, the defect viewing interface 1400A can also display the model information interface 1408, as well as other content.

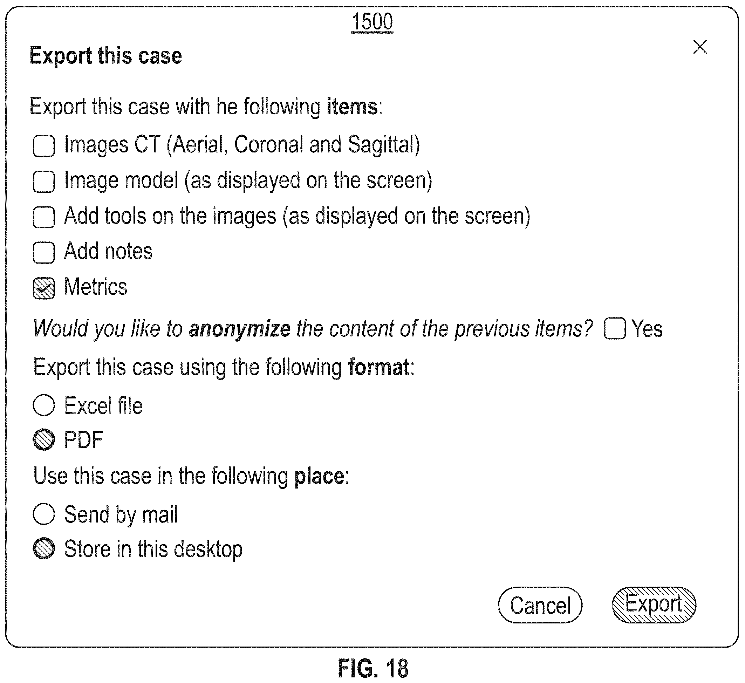

FIG. 18 depicts a case export interface 1500 of a surgical planning tool 140 according to one or more aspects. The case export interface 1500 can allow a user to select various items for export, such as scan images, model images, image overlays (e.g., measurements and/or annotations), notes, metrics, and other such information. The case export interface 1500 can include an anonymization option that removes or obfuscates personal identifying information from content being exported. The case export interface 1500 can also provide export format select (e.g., spreadsheet, document, etc.), export destination selection, and/or other such functions.

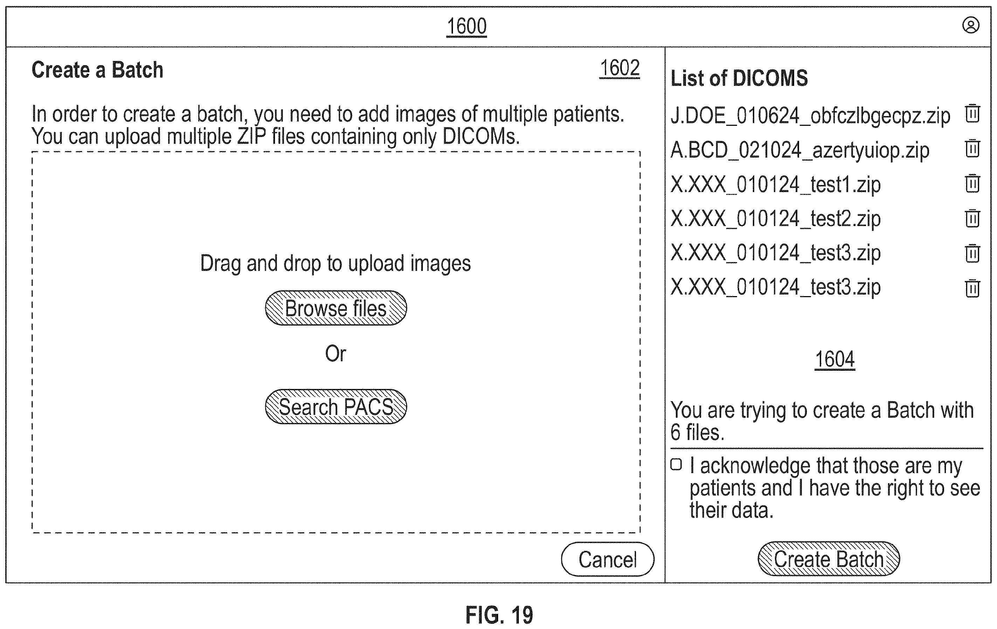

FIG. 19 depicts a batch creation interface 1600 of a surgical planning tool 140 according to one or more aspects. The batch creation interface 1600 can include a user interface 1602 to upload images and/or other related case information. Uploads can include file selection, drag-drop placement, or searching (e.g., searching a picture archiving and communication system (PACS) for content). The batch creation interface 1600 can include a list selection interface 1604 that lists content for inclusion in a batch. As an example, each item in the list can be a compressed file of image data, such as DICOM compatible data or other standards known in the art. The list selection interface 1604 can support adding, removing, or editing list content, as well as triggering batch creation.

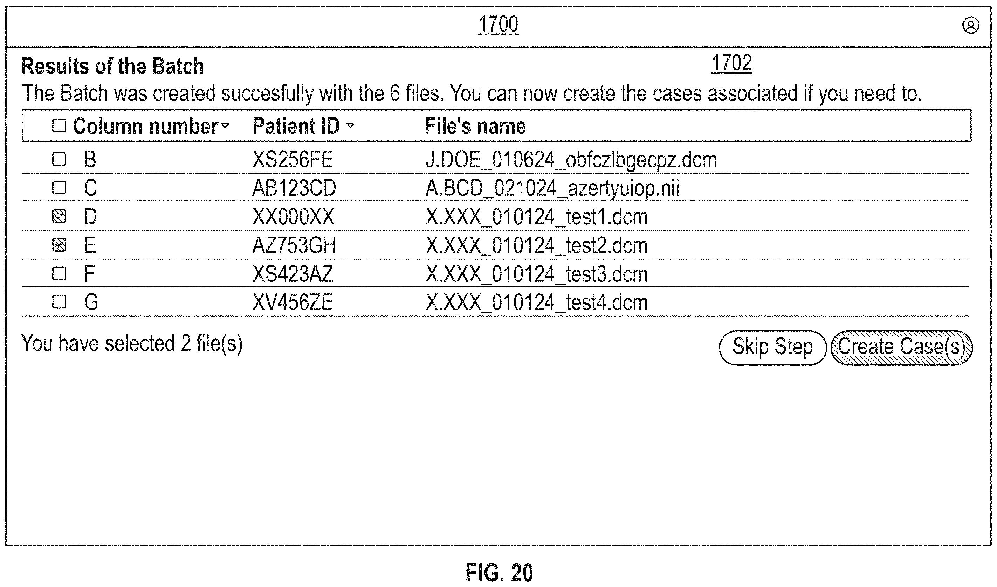

FIG. 20 depicts a batch results interface 1700 of a surgical planning tool 140 according to one or more aspects. The batch results interface 1700 can include a batch results list 1702 that summarizes import data as part of a batch and allows for case creation based on one or more selected files. For example, each line item can be associated with a different patient, including multiple images and related information per patient. The batch results interface 1700 can allow for faster selection of patient content for case creation. Batched data can also be accessed by the surgical planning tool 140 and used to perform metrics processing to support analysis across a group of patients without requiring individually opening and navigating through each case.

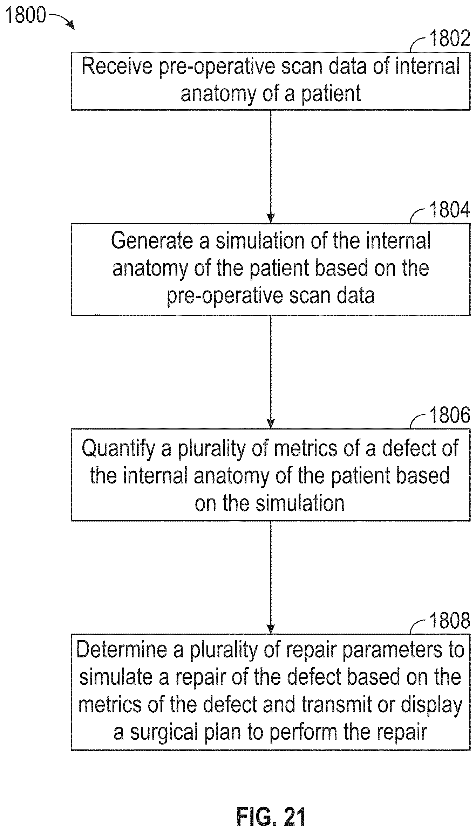

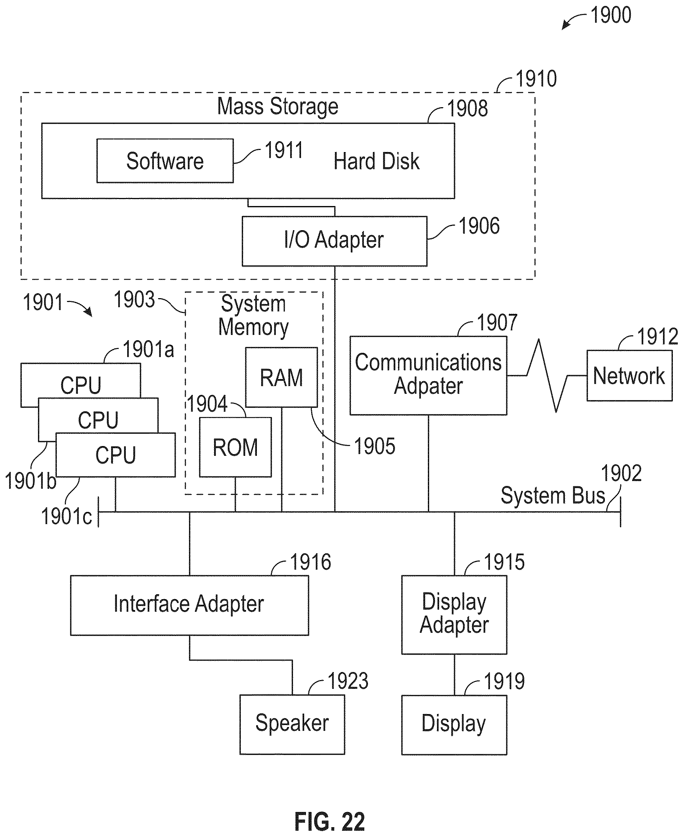

Turning now to FIG. 21, a flowchart of a method 1800 for a surgical planning tool is generally shown in accordance with one or more aspects. All or a portion of method 1800 can be implemented, for example, using aspects of the CAS system 100 of FIG. 1, the surgical procedure system 200 of FIG. 2, and/or computer system 1900 of FIG. 22.

At block 1802, the surgical planning tool 140 can receive pre-operative scan data of internal anatomy of a patient 110. At block 1804, the surgical planning tool 140 can generate a simulation of the internal anatomy of the patient based on the pre-operative scan data. At block 1806, the surgical planning tool 140 can provide an interactive digital measurement interface to quantify a plurality of metrics of a defect of the internal anatomy of the patient 110 based on the simulation. The defect can be, for example, a hernia. At block 1808, the surgical planning tool 140 can determine a plurality of repair parameters 1103 to simulate a repair of the defect based on the metrics of the defect and transmit or display a surgical plan to perform the repair. The repair can be a repair of a hernia using a mesh and a plurality of fixations based on the metrics of the hernia and the internal anatomy. The surgical plan can include positioning the mesh within the internal anatomy and applying the fixations. The surgical plan can establish simulated aspects of the repair and associated anatomy which can subsequently be used during a surgical procedure.

In some aspects, the surgical plan information can be shared with other systems to facilitate related actions, such as scheduling, inventory management, and other such actions. Surgical planning information can be used to estimate a surgery time (e.g., by surgical planning tool 140) and initiate a request to a hospital system to book resources accordingly. For example, surgical plan information can be transmitted to a hospital/center system that plans the operating room occupation and/or check the stock of inventory to make sure that the devices/implants/equipment needed will be available or if they should be ordered if out-of-stock.

The processing shown in FIG. 21 is not intended to indicate that the operations are to be executed in any particular order or that all of the operations shown in FIG. 21 are to be included in every case. Additionally, the processing shown in FIG. 21 can include any suitable number of additional operations.

In some aspects, a plurality of views 502 of the internal anatomy of the patient 110 can be displayed in an image viewing interface 500 of the surgical planning tool 140. At least one measurement of the hernia can be displayed in at least two of the views 502 in the image viewing interface 500.

In some aspects, the surgical planning tool 140 can generate a three-dimensional view of the internal anatomy of the patient 110 based on the views 602 of the internal anatomy. The at least one measurement of the hernia can be displayed in the three-dimensional view through a three-dimensional image viewing interface 600 of the surgical planning tool 140.

In some aspects, the surgical planning tool 140 can display the metrics in a metrics display 703 of a defect viewing interface 700 proximate to a view 702 of the internal anatomy, where the metrics can include one or more of: a hernia sac volume, a defect size, a hernia-neck ratio, a hernia defect angle, and a ratio defect width. The metrics may also include one or more of: a volume ratio of fat to abdominal cavity, a fat measurement, a muscle thickness, and an abdominal wall curvature.

In some aspects, receiving pre-operative scan data of internal anatomy of the patient 110 can include internal imaging in an at-rest state and an expanded state. The internal imaging can be displayed in the at-rest state proximate to the expanded state in a view 802 of a modified defect viewing interface 800 of the surgical planning tool 140. The surgical planning tool 140 can also display metrics 803 including one or more of: a hernia volume change, a critical resistance to impacts related to pressure value, a defect size change, and a defect stretch measurement based on at least one difference between the at-rest state and the expanded state.

In some aspects, the surgical planning tool 140 can display a component separation viewing interface 900 that depicts a simulation 902 of a transversus abdominis muscle release (TAR) relative to a baseline in combination with one or more charts 903 illustrating a simulated effect of performing the TAR and not performing the TAR.

In some aspects, the surgical planning tool 140 can display a repair sizing interface 1000 of a surgical planning tool 140 including a graphical overlay on a simulation 1002 illustrating sizing of the mesh relative to the internal anatomy and a mesh sizing interface 1003 including one or more of: a mesh size, a resulting overlap, a mesh-defect area ratio, and an indication of whether the mesh fits in a retro-rectus space of the internal anatomy.

In some aspects, the surgical plan can display a simulation interface 1100 including a simulation 1102 of stresses placed on the mesh for breathing and coughing conditions in combination with a comparison of a critical resistance to impacts related to pressure value to a gained resistance to impacts related to pressure value for the mesh with the fixations applied as part of repair parameters 1103.

Turning now to FIG. 22, a computer system 1900 is generally shown in accordance with an aspect. The computer system 1900 can be an electronic computer framework comprising and/or employing any number and combination of computing devices and networks utilizing various communication technologies, as described herein. The computer system 1900 can be easily scalable, extensible, and modular, with the ability to change to different services or reconfigure some features independently of others. The computer system 1900 may be, for example, a server, desktop computer, laptop computer, tablet computer, or smartphone. In some examples, computer system 1900 may be a cloud computing node. Computer system 1900 may be described in the general context of computer-executable instructions, such as program modules, being executed by a computer system. Generally, program modules may include routines, programs, objects, components, logic, data structures, and so on that perform particular tasks or implement particular abstract data types. Computer system 1900 may be practiced in distributed cloud computing environments where tasks are performed by remote processing devices that are linked through a communications network. In a distributed cloud computing environment, program modules may be located in both local and remote computer system storage media, including memory storage devices.

As shown in FIG. 22, the computer system 1900 has a processing system 1901 including one or more central processing units (CPU(s)) 1901a, 1901b, 1901c, etc. (collectively or generically referred to as processor(s) 1901). The processors 1901 can be a single-core processor, multi-core processor, computing cluster, or any number of other configurations. The processors 1901 can be any type of circuitry capable of executing instructions. The processors 1901, also referred to as processing circuits, are coupled via a system bus 1902 to a system memory 1903 and various other components. The system memory 1903 can include one or more memory devices, such as read-only memory (ROM) 1904 and a random-access memory (RAM) 1905. The ROM 1904 is coupled to the system bus 1902 and may include a basic input/output system (BIOS), which controls certain basic functions of the computer system 1900. The RAM is read-write memory coupled to the system bus 1902 for use by the processors 1901. The system memory 1903 provides temporary memory space for operations of said instructions during operation. The system memory 1903 can include random access memory (RAM), read-only memory, flash memory, or any other suitable memory systems.

The computer system 1900 comprises an input/output (I/O) adapter 1906 and a communications adapter 1907 coupled to the system bus 1902. The I/O adapter 1906 may be a small computer system interface (SCSI) adapter that communicates with a hard disk 1908 and/or any other similar component. The I/O adapter 1906 and the hard disk 1908 are collectively referred to herein as a mass storage 1910.

Software 1911 for execution on the computer system 1900 may be stored in the mass storage 1910. The mass storage 1910 is an example of a tangible storage medium readable by the processors 1901, where the software 1911 is stored as instructions for execution by the processors 1901 to cause the computer system 1900 to operate, such as is described hereinbelow with respect to the various Figures. Examples of computer program product and the execution of such instruction is discussed herein in more detail. The communications adapter 1907 interconnects the system bus 1902 with a network 1912, which may be an outside network, enabling the computer system 1900 to communicate with other such systems. In one aspect, a portion of the system memory 1903 and the mass storage 1910 collectively store an operating system, which may be any appropriate operating system to coordinate the functions of the various components shown in FIG. 22.

Additional input/output devices are shown as connected to the system bus 1902 via a display adapter 1915 and an interface adapter 1916 and. In one aspect, the adapters 1906, 1907, 1915, and 1916 may be connected to one or more I/O buses that are connected to the system bus 1902 via an intermediate bus bridge (not shown). A display 1919 (e.g., a screen or a display monitor) is connected to the system bus 1902 by a display adapter 1915, which may include a graphics controller to improve the performance of graphics-intensive applications and a video controller. A keyboard, a mouse, a touchscreen, one or more buttons, a speaker, etc., can be interconnected to the system bus 1902 via the interface adapter 1916, which may include, for example, a Super I/O chip integrating multiple device adapters into a single integrated circuit. Suitable I/O buses for connecting peripheral devices such as hard disk controllers, network adapters, and graphics adapters typically include common protocols, such as the Peripheral Component Interconnect (PCI). Thus, as configured in FIG. 22, the computer system 1900 includes processing capability in the form of the processors 1901, and storage capability including the system memory 1903 and the mass storage 1910, input means such as the buttons, touchscreen, and output capability including the speaker 1923 and the display 1919.

In some aspects, the communications adapter 1907 can transmit data using any suitable interface or protocol, such as the internet small computer system interface, among others. The network 1912 may be a cellular network, a radio network, a wide area network (WAN), a local area network (LAN), or the Internet, among others. An external computing device may connect to the computer system 1900 through the network 1912. In some examples, an external computing device may be an external web server or a cloud computing node.

It is to be understood that the block diagram of FIG. 22 is not intended to indicate that the computer system 1900 is to include all of the components shown in FIG. 22. Rather, the computer system 1900 can include any appropriate fewer or additional components not illustrated in FIG. 22 (e.g., additional memory components, embedded controllers, modules, additional network interfaces, etc.). Further, the aspects described herein with respect to computer system 1900 may be implemented with any appropriate logic, wherein the logic, as referred to herein, can include any suitable hardware (e.g., a processor, an embedded controller, or an application-specific integrated circuit, among others), software (e.g., an application, among others), firmware, or any suitable combination of hardware, software, and firmware, in various aspects. Various aspects can be combined to include two or more of the aspects described herein.

In some aspects, a system can include a memory system 1903 and a processing system 1901 coupled to the memory system 1903 and configured to execute instructions to perform a plurality of operations including receiving, by a surgical planning tool 140, pre-operative scan data of internal anatomy of a patient 110, the pre-operative scan data including data from one or more scans from an imaging system 104. The operations can also include generating, by the surgical planning tool 140, a simulation of the internal anatomy of the patient 110 based on the pre-operative scan data by combining the data from two or more views 502, 602 of the one or more scans; quantifying, by the surgical planning tool 140, a plurality of metrics of a defect of the internal anatomy of the patient 110 based on the simulation; determining, by the surgical planning tool 140, a plurality of repair parameters 1103 to simulate a repair of the defect based on the metrics of the defect; and displaying, by the surgical planning tool 140, a surgical plan to perform the repair.

In some aspects, the two or more views 502, 602 of the one or more scans can include two or more of: a front view, a rear view, a side view, and an axial view.

In some aspects, upon execution, the instructions can be configured to perform the operations including providing a batch processing interface to process a group of data associated with two of more cases and performing metrics processing on the two or more cases based on a selection of the group. The batch processing interface can include one or more of the case selection interface 1200, the batch creation interface 1600, and the batch results interface 1700.

In some aspects, upon execution, the instructions can be configured to perform the operations including generating, by the surgical planning tool 140, a three-dimensional rendering of the internal anatomy based on a machine-learning model 330 trained to project the data from the one or more scans into three-dimensional space and identify a plurality of anatomical features.

In some aspects, the processing system 1901 can be configured to communicate with a surgical procedure support system 202 to provide the surgical plan for use during the surgical procedure.

In some aspects, the processing system 1901 can be configured to receive feedback from the surgical procedure support system 202 to document an outcome of the surgical procedure.

In some aspects, displaying the surgical plan to perform the repair can include displaying an animation illustrating one or more stress impacts associated with the repair.

A computer program product can include a memory device having computer executable instructions stored thereon, which when executed by one or more processors 1901 cause the one or more processors 1901 of a computing system to perform a plurality of operations. The operations can include receiving, by a surgical planning tool 140, pre-operative scan data of internal anatomy of a patient 110; generating, by the surgical planning tool 140, a simulation of the internal anatomy of the patient 110 based on the pre-operative scan data; quantifying, by the surgical planning tool 140, a plurality of metrics of a defect of the internal anatomy of the patient 110 based on the simulation; determining, by the surgical planning tool 140, a plurality of repair parameters 1103 to simulate a repair of the defect based on the metrics of the defect; and displaying a surgical plan to perform the repair.

In some aspects, the simulation is displayed in three-dimensions with color coded anatomical features.

In some aspects, a interactive digital measurement interface can provide one or more of: a linear measurement, an area measurement, a volume measurement, an angle measurement, a curvature measurement, and a ratio measurement.

In some aspects, the operations can include displaying, by the surgical planning tool 140, one or more recommendations to modify one or more of the repair parameters 1103 based on comparing the metrics of the defect and the one or more of the repair parameters 1103 to one or more performance criteria.

In some aspects, the operations can include recomputing the one or more of the repair parameters 1103 based on a change of the surgical plan and displaying a simulated effect based on the change to the surgical plan.

Aspects disclosed herein may be a system, a method, and/or a computer program product at any possible technical detail level of integration. The computer program product may include a computer-readable storage medium (or media) having computer-readable program instructions thereon for causing a processor to carry out various aspects.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device, such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network, and/or a wireless network. The network may comprise copper transmission cables, optical transmission fibers, wireless transmission, routers, firewalls, switches, gateway computers, and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present disclosure may be assembler instructions, instruction-set-architecture (ISA) instructions, machine instructions, machine-dependent instructions, microcode, firmware instructions, state-setting data, configuration data for integrated circuitry, or either source-code or object code written in any combination of one or more programming languages, including an object-oriented programming language, such as Smalltalk, C++, high-level languages such as Python, or the like, and procedural programming languages, such as the "C" programming language or similar programming languages. The computer-readable program instructions may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some aspects, electronic circuitry including, for example, programmable logic circuitry, field-programmable gate arrays (FPGA), or programmable logic arrays (PLA) may execute the computer-readable program instruction by utilizing state information of the computer-readable program instructions to personalize the electronic circuitry, in order to perform aspects of the present disclosure.

Aspects are described herein with reference to flowchart illustrations and/or block diagrams of methods, apparatus (systems), and computer program products according to aspects of the disclosure. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a computer system, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other devices to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various aspects. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various aspects have been presented for purposes of illustration but are not intended to be exhaustive or limited to the aspects disclosed. Many modifications and variations will be apparent to those of ordinary skill in the art without departing from the scope and spirit of the described aspects. The terminology used herein was chosen to best explain the principles of the aspects, the practical application or technical improvement over technologies found in the marketplace, or to enable others of ordinary skill in the art to understand the aspects described herein.

Various aspects are described herein with reference to the related drawings. Alternative aspects can be devised without departing from the scope of this disclosure. Various connections and positional relationships (e.g., over, below, adjacent, etc.) are set forth between elements in the following description and in the drawings. These connections and/or positional relationships, unless specified otherwise, can be direct or indirect, and the present disclosure is not intended to be limiting in this respect. Accordingly, a coupling of entities can refer to either a direct or an indirect coupling, and a positional relationship between entities can be a direct or indirect positional relationship. Moreover, the various tasks and process steps described herein can be incorporated into a more comprehensive procedure or process having additional steps or functionality not described in detail herein.

The following definitions and abbreviations are to be used for the interpretation of the claims and the specification. As used herein, the terms "comprises," "comprising," "includes," "including," "has," "having," "contains," or "containing," or any other variation thereof are intended to cover a non-exclusive inclusion. For example, a composition, a mixture, process, method, article, or apparatus that comprises a list of elements is not necessarily limited to only those elements but can include other elements not expressly listed or inherent to such composition, mixture, process, method, article, or apparatus.

Additionally, the term "exemplary" is used herein to mean "serving as an example, instance or illustration." Any aspect or design described herein as "exemplary" is not necessarily to be construed as preferred or advantageous over other aspects or designs. The terms "at least one" and "one or more" may be understood to include any integer number greater than or equal to one, i.e., one, two, three, four, etc. The terms "a plurality" may be understood to include any integer number greater than or equal to two, i.e., two, three, four, five, etc. The term "connection" may include both an indirect "connection" and a direct "connection."

The terms "about," "substantially," "approximately," and variations thereof are intended to include the degree of error associated with measurement of the particular quantity based upon the equipment available at the time of filing the application. For example, "about" can include a range of ± 8% or 5%, or 2% of a given value.

For the sake of brevity, conventional techniques related to making and using aspects may or may not be described in detail herein. In particular, various aspects of computing systems and specific computer programs to implement the various technical features described herein are well known. Accordingly, in the interest of brevity, many conventional implementation details are only mentioned briefly herein or are omitted entirely without providing the well-known system and/or process details.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium, such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), graphics processing units (GPUs), microprocessors, application-specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A computer-implemented method comprising:
receiving, by a surgical planning tool (140), pre-operative scan data of internal anatomy of a patient (110);
generating, by the surgical planning tool (140), a simulation of the internal anatomy of the patient (110) based on the pre-operative scan data;
quantifying, by the surgical planning tool (140), a plurality of metrics of a hernia and the internal anatomy of the patient (110) based on the simulation;
determining, by the surgical planning tool (140), a plurality of repair parameters (1103) to simulate a repair of the hernia using a mesh and a plurality of fixations based on the metrics of the hernia and the internal anatomy; and
displaying, by the surgical planning tool (140), a surgical plan to perform the repair by positioning the mesh within the internal anatomy and applying the fixations.

2. The computer-implemented method of claim 1, further comprising:
displaying a plurality of views (502) of the internal anatomy of the patient (110) in an image viewing interface (500) of the surgical planning tool (140); and
displaying at least one measurement of the hernia in at least two of the views (502) in the image viewing interface (500).

3. The computer-implemented method of claims 1 or 2, further comprising:
generating a three-dimensional view of the internal anatomy of the patient (110) based on a plurality of views (602) of the internal anatomy; and
displaying the at least one measurement of the hernia in the three-dimensional view through a three-dimensional image viewing interface (600) of the surgical planning tool (140).

4. The computer-implemented method of any of claims 1 to 3, further comprising:
displaying the metrics in a metrics display (703) of a defect viewing interface (700) of the surgical planning tool (140) proximate to a view (702) of the internal anatomy, wherein the metrics comprise one or more of: a hernia sac volume, a defect size, a hernia-neck ratio, a hernia defect angle, and a ratio defect width; and optionally, wherein the metrics comprise one or more of: a volume ratio of fat to abdominal cavity, a fat measurement, a muscle thickness, and an abdominal wall curvature.

5. The computer-implemented method of any of claims 1 to 4, wherein receiving pre-operative scan data of internal anatomy of the patient (110) comprises internal imaging in an at-rest state and an expanded state, and further comprising:
displaying the internal imaging in the at-rest state proximate to the expanded state in a view (802) of a modified defect viewing interface (800) of the surgical planning tool (140); and
displaying metrics (803) comprising one or more of: a hernia volume change, a critical resistance to impacts related to pressure value, a defect size change, and a defect stretch measurement based on at least one difference between the at-rest state and the expanded state.

6. The computer-implemented method of any of claims 1 to 5, further comprising:
displaying a component separation viewing interface (900) of the surgical planning tool (140) that depicts a simulation (902) of a transversus abdominis muscle release (TAR) relative to a baseline in combination with one or more charts (903) illustrating a simulated effect of performing the TAR and not performing the TAR.

7. The computer-implemented method of any of claims 1 to 6, further comprising:
displaying a repair sizing interface (1000) of the surgical planning tool (140) comprising a graphical overlay on a simulation (1002) illustrating sizing of the mesh relative to the internal anatomy and a mesh sizing interface (1003) comprising one or more of: a mesh size, a resulting overlap, a mesh-defect area ratio, and an indication of whether the mesh fits in a retro-rectus space of the internal anatomy.

8. The computer-implemented method of any of claims 1 to 7, wherein displaying the surgical plan comprises displaying a simulation interface (1100) including a simulation (1102) with of stresses placed on the mesh for breathing and coughing conditions in combination with a comparison of a critical resistance to impacts related to pressure value to a gained resistance to impacts related to pressure value for the mesh with the fixations applied as part of the repair parameters (1103).

9. A system comprising:
a memory system (1303) ; and
a processing system (1301) coupled to the memory system (1303) and configured to execute instructions to perform a plurality of operations comprising:
receiving, by a surgical planning tool (140), pre-operative scan data of internal anatomy of a patient (110), the pre-operative scan data comprising data from one or more scans from an imaging system (104);
generating, by the surgical planning tool (140), a simulation of the internal anatomy of the patient (110) based on the pre-operative scan data by combining the data from two or more views (502, 602) of the one or more scans;
quantifying, by the surgical planning tool (140), a plurality of metrics of a defect of the internal anatomy of the patient based on the simulation;
determining, by the surgical planning tool (140), a plurality of repair parameters (1103) to simulate a repair of the defect based on the metrics of the defect; and
transmitting, by the surgical planning tool (140), a surgical plan to perform the repair.

10. The system of claim 9, wherein upon execution, the instructions are configured to perform the operations comprising:
providing a batch processing interface to process a group of data associated with two of more cases; and
performing metrics processing on the two or more cases based on a selection of the group.

11. The system of claims 9 or 10, wherein upon execution, the instructions are configured to perform the operations comprising:
generating, by the surgical planning tool (140), a three-dimensional rendering of the internal anatomy based on a machine-learning model (330) trained to project the data from the one or more scans into three-dimensional space and identify a plurality of anatomical features.

12. The system of any of claims 9 to 11, wherein the processing system (1301) is configured to communicate with a surgical procedure support system (202) to provide the surgical plan for use during the surgical procedure, and optionally, wherein the processing system (1301) is configured to receive feedback from the surgical procedure support system (202) to document an outcome of the surgical procedure.

13. The system of any of claims 9 to 12, wherein displaying the surgical plan to perform the repair comprises displaying an animation illustrating one or more stress impacts associated with the repair.

14. A computer program product comprising a memory device having computer executable instructions stored thereon, which when executed by one or more processors (1301) cause the one or more processors (1301) of a computing system to perform a plurality of operations comprising:
receiving, by a surgical planning tool (140), pre-operative scan data of internal anatomy of a patient (110);
generating, by the surgical planning tool (140), a simulation of the internal anatomy of the patient (110) based on the pre-operative scan data;
quantifying, by the surgical planning tool (140), a plurality of metrics of a defect of the internal anatomy of the patient based on the simulation;
determining, by the surgical planning tool (140), a plurality of repair parameters (1103) to simulate a repair of the defect based on the metrics of the defect; and
displaying a surgical plan to perform the repair.

15. The computer program product of claim 14, wherein the computer executable instructions, which when executed by the one or more processors (1301) cause the one or more processors (1301) to perform the operations comprising:
displaying, by the surgical planning tool (140), one or more recommendations to modify one or more of the repair parameters (1103) based on comparing the metrics of the defect and the one or more of the repair parameters (1103) to one or more performance criteria, and optionally, recomputing the one or more of the repair parameters (1103) based on a change of the surgical plan and displaying a simulated effect based on the change to the surgical plan.
